# EUROPEAN PATENT SPECIFICATION

(11) **EP 2 995 321 B1**
(45) Date of publication and mention of the grant of the patent: **26.07.2017**
(21) Application number: 15161889.9
(22) Date of filing: 31.03.2015
(51) Int. Cl.: A61L 15/00, A61K 31/722, A61Q 19/00, C08L 97/00

(54) **A CONSUMER GOODS PRODUCT COMPRISING CHITIN NANOFIBRILS, LIGNIN AND A POLYMER OR CO-POLYMER**
KONSUMGÜTERPRODUKT MIT CHITIN NANOFIBRILLEN, LIGNIN UND EINEM POLYMER ODER CO-POLYMER
PRODUIT DE BIENS DE CONSOMMATION CONTENANT DES NANOFIBRILLES DE CHITINE, DE LA LIGNINE ET UN POLYMÈRE OU COPOLYMÈRE

(30) Priority: 15.09.2014 IT RM20140523
(43) Date of publication of application: 16.03.2016
(73) Proprietor: Procter & Gamble International Operations SA, 1213 Geneva (CH); Mavi Sud S.R.L, 04011 Aprilia LT (IT)
(72) Inventor: Brooker, Anju Deepali Massey, Newcastle upon Tyne, Tyne and Wear NE12 9TS (GB); Vaccaro, Mauro, Newcastle upon Tyne, Northumberland NE12 9TS (GB); Scialla, Stefano, 0040 Pomezia (IT); Walker, Stephen John, Newcastle-upon-Tyne, NE12 9TS (GB); Morganti, Pierefrancesco, 04011 Aprilia (IT); Carezzi, Francesco, 04011 Aprilia (IT); Benjelloun-Mlayah, Bouchra, 92200 Neuilly-sur-Seine (FR); Crestini, Claudia, 00173 Rome (IT); Lange, Heiko, 00173 Rome (IT); Bartzoka, Elisavet, 00173 Rome (IT)
(74) Representative: Howard, Phillip Jan

(56) References cited:
- WO-A1-2007/060628
- WO-A1-2012/138802
- WO-A1-2012/143875

## Description

### FIELD OF THE INVENTION

The present invention relates to consumer goods products comprising chitin nanofibrils, lignin or a derivative thereof, and optionally at least one polymer or co-polymer, to a process for their preparation.

### BACKGROUND OF THE INVENTION

In recent years, many efforts have been made towards the development of materials for medical, cosmetic and biological purposes, in particular for the development of polymer products suitable for the treatment of inflammation and tissue regeneration and in regard to cellular technology and organ transplantation. The key characteristics that these materials must possess are biocompatibility and biodegradability both as regards to the constituent polymers and to the products of their decomposition. In particular, polyglycols, polyacids, polylactones, polysaccharides and other natural and synthetic polymers are used for the preparation of bioabsorbable matrix (Langer R., Tirrell DA Designing materials for biology and medicine // Nature, 2004. Vol. 428, 6982, pp. 487-492).

Films, fabrics formed by interlacing irregular micro/nanofibres (non-woven tissues) and porous materials based primarily on the use of natural chitin polysaccharide (CN) (Pillai C.K.S., Paul W., Sharma C. P. Chitin and chitosan polymers have been recently used as matrix for biomedical applications: Chemistry, solubility and fiber formation // Progress in Polymer Science. 2009. Vol. 34. P. 641-678). Due to its non-toxic nature and given that it is derived from the processing of fish and shellfish waste without depleting the environment of valuable raw materials, the use of this natural polymer is increasing year on year.

In particular, due to its interesting properties such as the absence of cytotoxicity, easy bioabsorption and the ecology of its preparation procedure, all of which encourage its use in the biomedical field, the N-deacetylated chitin derivative, i.e. chitosan (CS), is amongst the most promising of the polymers. However, because of its hydrophilic nature, CS-based articles are unstable, fragile, not particularly elastic and substantially rigid in the wet state.

The object of the present invention is to develop consumer goods products comprising a suitable chitin/lignin material that can be used to provide benefits to a range of surfaces.

The consumer goods products of the present invention provide anti-inflammation benefits and anti-oxidant benefits to skin and/or gum, for example in feminine pads, diapers, razor blade strips, teeth whitening strips, skin creams, skin lotions shaving preparation gels or foams, handwash laundry detergents, handwash dishwashing detergents, soap bars, liquid handwash soap, body wash, toothpastes, shampoo, and conditioners.

In addition, the consumer goods products of the present invention provide excellent deposition of active benefits to a range of surfaces, including ceramics, glass and skin. Suitable actives include aloe vera, zinc, silicone including terminal amino silicone, shea butter, choline salicylate, zinc carbonate, terpene, limonene, monoethyleneamine, peroxide including di-acyl peroxide and/or di-benyl peroxide, silver, blood coaggulant, titanium dioxide, benzo triazole, sodium dichloroisocyanurate, perfume, sense aids, and any mixture thereof.

The consumer goods product may also be a hard surface cleaning sheet and/or wipe.

### SUMMARY OF THE INVENTION

The present invention provides a consumer goods product comprising a composition comprising or consisting of chitin nanofibrils, lignin or its derivatives and optionally at least one polymer or co-polymer.

### DETAILED DESCRIPTION OF THE INVENTION

**Consumer goods product:** The present invention provides a consumer goods product comprising a composition comprising or consisting of chitin nanofibrils, lignin or its derivatives and optionally at least one polymer or co-polymer.

The consumer goods product is typically selected from: feminine pad; diaper; razor blade strip; hard surface cleaning sheet and/or wipe; and teeth treatment strip.

The consumer goods product is typically selected from: skin cream; skin lotion; shaving preparation gel or foam; handwash laundry detergent; handwash dishwashing detergent; soap bar; liquid handwash soap; body wash; toothpaste; shampoo; and conditioner.

The consumer goods product may be a cleaning sheet. The cleaning sheet according to the present invention may comprise a nonwoven. The nonwoven may be synthetic and/or have cellulosic fibers therein. The synthetic fibers may comprise carded, staple, wet laid, air laid and/or spunbond fibers. The nonwoven cleaning sheet may be made according to a hydroentangling process to provide a texture and a basis weight of about 20 to about 120 gsm.

Optionally, the cleaning sheet may further comprise an additive, to improve cleaning performance and/or enhance the cleaning experience. The additive may comprise wax, such as microcrystalline wax, oil, adhesive, perfume and combinations thereof.

The cleaning sheet according to the present invention may be made according to commonly assigned US patents 6,305,046; 6,484,346; 6,561,354; 6,645,604; 6,651,290; 6,777,064; 6,790,794; 6,797,357**;** 6,936,330**;** D409,343; D423,742; D489,537; D498,930; D499,887; D501,609; D511,251 and/or D615,378.

If desired, the cleaning sheet may be pre-moistened. If the cleaning sheet is pre-moistened, it is preferably pre-moistened with a liquid which provides for cleaning of the target surface, such as a floor, but yet does not require a post-cleaning rinsing operation.

The pre-moistened cleaning sheet may comprise natural or synthetic fibers. The fibers may be hydrophillic, hydrophobic or a combination thereof, provided that the cleaning sheet is generally absorbent to hold, and express upon demand, a cleaning solution. In one embodiment, the cleaning sheet may comprise at least 50 weight percent or at least 70 weight percent cellulose fibers, such as air laid SSK fibers. If desired, the cleaning sheet may comprise plural layers to provide for scrubbing, liquid storage, and other particularized tasks for the cleaning operation.

The cleaning sheet may be loaded with at least 1, 1.5 or 2 grams of cleaning solution per gram of dry substrate, but typically not more than 5 grams per gram. The cleaning solution may comprise a surfactant, such as APG surfactant which minimizes streaking since there is typically not a rinsing operation, agglomerating chemicals, disinfectants, bleaching solutions, perfumes, secondary surfactants etc.

Optionally, the pre-moistened cleaning sheet may further comprise a scrubbing strip. A scrubbing strip is a portion of the cleaning sheet which provides for more aggressive cleaning of the target surface. A suitable scrubbing strip may comprise a polyolefinic film, such as LDPE, and have outwardly extending perforations, etc. The scrubbing strip may be made and used according to commonly assigned US patents 8,250,700; 8,407,848; D551,409 S and/or D614,408 S.

A suitable pre-moistened cleaning sheet maybe made according to the teachings of commonly assigned US patents 6,716,805**;** D614,408; D629,211 and/or D652,633.

The cleaning sheet according to the present invention may be used with a stick-type cleaning implement. The cleaning implement may comprise a plastic head for holding the cleaning sheet and an elongate handle articulably connected thereto. The handle may comprise a metal or plastic tube or solid rod.

The head may have a downwardly facing surface, to which the sheet may be attached. The downwardly facing service may be generally flat, or slightly convex. The head may further have an upwardly facing surface. The upwardly facing surface may have a universal joint to facilitate connection of the elongate handle to the head.

The upwardly facing surface may further comprise a mechanism, such as resilient grippers, for removably attaching the cleaning sheet to the implement. Alternatively, a hook and loop system may be used to attach the cleaning sheet to the head. If grippers are used with the cleaning implement, the grippers may be made according to commonly assigned US patents 6,305,046; 6,484,346**;** 6,651,290 and/or D487,173.

If desired, the cleaning implement may have an axially rotatable beater bar and/or vacuum type suction to assist in removal of debris from the target surface. Debris removed from the target surface may be collected in a dust bin. The dust bin may be mounted within the head, or, alternatively, on the elongate handle.

A suitable stick-type cleaning implement may be made according to commonly assigned US patents Des. 391,715; D409,343; D423,742; D481,184; D484,287; D484,287 and/or D588,770. A suitable vacuum type cleaning implement may be made according to the teachings of US patents 7,137,169**,** D484,287 S, D615,260 S and D615,378 S. An implement having a beater bar may be made according to commonly assigned US 2013/0333129. A motorized implement may be made according to commonly assigned US patent 7,516,508.

The cleaning sheet according to the present invention may comprise a nonwoven. The nonwoven may be synthetic and/or have cellulosic fibers therein. The synthetic fibers may comprise carded, staple, wet laid, air laid and/or spunbond fibers.

The cleaning sheet may comprise layers, to provide for absorption and storage of cleaning fluid deposited on the target surface. If desired, the cleaning sheet may comprise absorbent gelling materials to increase the absorbent capacity of the cleaning sheet. The absorbent gelling materials may be distributed within the cleaning sheet in such a manner to avoid rapid absorbency and absorb fluids slowly, to provide for the most effective use of the cleaning sheet.

The cleaning sheet may comprise plural layers disposed in a laminate. The lowest, or downwardly facing outer layer, may comprise apertures to allow for absorption of cleaning solution therethrough and to promote the scrubbing of the target surface. Intermediate layers may provide for storage of the liquids, and may comprise the absorbent gelling materials. The cleaning sheet may have an absorbent capacity of at least 10, 15, or 20 grams of cleaning solution per gram of dry cleaning sheet, as set forth in commonly assigned US Patent 6,003,191 and 6,601,261**.**

The top,or upwardly facing outer layer, maybe liquid impervious in order to minimize loss of absorbed fluids. The top layer may further provide for releasable attachment of the cleaning sheet to a cleaning implement. The top layer may be made of a polyolefinic film, such as LDPE.

The cleaning sheet according to the present invention may be used with a cleaning implement. The cleaning implement may comprise a plastic head for holding the cleaning sheet and an elongate handle articulably connected thereto. The handle may comprise a metal or plastic tube or solid rod.

The head may have a downwardly facing surface, to which the sheet may be attached. The downwardly facing service may be generally flat, or slightly convex. The head may further have an upwardly facing surface. The upwardly facing surface may have a universal joint to facilitate connection of the elongate handle to the head.

A hook and loop system may be used to attach the cleaning sheet directly to the bottom of the head. Alternatively, the upwardly facing surface may further comprise a mechanism, such as resilient grippers, for removably attaching the cleaning sheet to the implement. If grippers are used with the cleaning implement, the grippers may be made according to commonly assigned US patents 6,305,046; 6,484,346; 6,651,290 and/or D487,173.

The cleaning implement may further comprise a reservoir for storage of cleaning solution. The reservoir may be replaced when the cleaning solution is depleted and/or refilled as desired. The reservoir may be disposed on the head or the handle of the cleaning implement. The neck of the reservoir may be offset per commonly assigned US patent 6,390,335. The cleaning solution contained therein may be made according to the teachings of commonly assigned US patent 6,814,088**.**

The cleaning implement may further comprise a pump for dispensing cleaning solution from the reservoir onto the target surface, such as a floor. The pump may be battery powered or operated by line voltage. Alternatively, the cleaning solution may be dispensed by gravity flow. The cleaning solution may be sprayed through one or more nozzles to provide for distribution of the cleaning solution onto the target surface in an efficacious pattern.

If a replaceable reservoir is utilized, the replaceable reservoir may be inverted to provide for gravity flow of the cleaning solution. Or the cleaning solution may be pumped to the dispensing nozzles. The reservoir may be a bottle, and may made of plastic, such as a polyolefin. The cleaning implement may have a needle to receive the cleaning solution from the bottle. The bottle may have a needle piercable membrane, complementary to the needle, and which is resealed to prevent undesired dripping of the cleaning solution during insertion and removal of the replaceable reservoir. Alternatively or additionally, If desired, the implement may also provide for steam to be delivered to the cleaning sheet and/or to the floor or other target surface.

A suitable reservoir and fitment therefor may be made according to the teachings of commonly assigned US Patents 6,386,392**,** 7,172,099; D388,705; D484,804; D485,178. A suitable cleaning implement may be made according to the teachings of commonly assigned US Patents 5,888,006; 5,960,508; 5,988,920; 6,045,622; 6,101,661; 6,142,750; 6,579,023**;** 6,601,261; 6,722,806; 6,766,552; D477,701 and/or D487,174. A steam implement may be made according to the teachings of jointly assigned 2013/0319463

The consumer goods product may be a cleaning article. The cleaning article according to the present invention may comprise a nonwoven sheet having tow fibers joined thereto. The cleaning article may have a longitudinal axis. The tow fibers may be joined to the nonwoven sheet in a generally transverse direction and particularly in a direction normal the longitudinal axis, to provide a laminate structure of two laminae.

If desired, the cleaning article may comprise additional laminae. For example, the tow fibers may be disposed intermediate two nonwoven sheets. Plural laminae of tow fibers may be disposed intermediate the nonwoven sheets and/or outboard thereof. Optionally, one or more of the nonwoven sheets may be cut to provide comprise strips. The strips may be generally normal to the longitudinal axis.

The tow fibers and/or nonwoven sheets may comprise an additive to assist in removal of dust and other debris from the target surface. The additive may comprise wax, such as microcrystalline wax, oil, adhesive and combinations thereof. The cleaning article may be made according to US Patent 6,813,801**.**

The laminae of the cleaning article may be joined together using adhesive, thermal bonding, ultrasonic welding, etc. If desired, the bonding lines may be generally parallel to the longitudinal axis and may be continuous, or discontinuous as desired. Three longitudinally parallel bonding lines may be utilized to define two sleeves.

The two sleeves may accept one or more complementary fork tines of a handle. The fork tines may be removably inserted into the sleeves of the cleaning article to provide for improved ergonomics. The handle may be plastic and made according to the teachings of US patents 7,219,386; 7,293,317 and/or 7,383,602.

The consumer goods product may be an absorbent article. Non-limiting examples of disposable absorbent articles include diapers, training pants, adult incontinence products, and feminine hygiene products (including, for example, sanitary napkins and tampons).

Absorbent article may comprise a chassis comprising a topsheet, a backsheet, and an absorbent core disposed at least partially between the topsheet and the backsheet. The absorbent chassis may comprise a waistband, leg cuffs and or elastic strands. Flaps comprising fastening components may be attached to or integral with the chassis. Exemplary taped and pant-style diapers, as well as pads and liner-type articles are disclosed U.S. Application No. 61/931,229.

A chassis of the absorbent article may comprise a topsheet. Suitable apertured films that may be used as or in combination with the topsheet are described in U.S. Pat. Nos. 3,929,135; 4,324,246; 4,342,314; 4,463,045; 5,006,394; 5,628,097; 5,916,661; 6,545,197; and 6,107,539.

Examples of suitable topsheet lotions include, but are not limited to, those described in U.S. Pat. Nos. 5,607,760; 5,609,587; 5,635,191; 5,643,588; and 5,968,025, and as described in U.S. Application No. 61/391,353.

The chassis of the absorbent article may comprise an absorbent core (often referred to as an absorbent assembly, structure, or member). In one embodiment, suitable absorbent cores may comprise cellulosic airfelt material. For instance, such absorbent cores may comprise less than about 40%, 30%, 20%, 10%, 5%, or even 1% of the cellulosic airfelt material as determined by weight. Additionally, such an absorbent core may be primarily comprised of an absorbent gelling material in amounts of at least about 60%, 70%, 80%, 85%, 90%, 95%, or even about 100% as determined by weight. Furthermore, a portion of the absorbent core may comprise a microfiber glue (if applicable). Such absorbent cores, microfiber glues, and absorbent gelling materials are described in U.S. Pat. Nos. 5,599,335; 5,562,646; 5,669,894; 6,790,798; and 7,521,587 and in U.S. Pat. Pub. No. 2004/0158212.

The chassis of the absorbent article may comprise leg cuffs. Suitable elasticized leg cuffs may comprise those described in U.S. Pat. Nos. 3,860,003; 4,909,803; 4,695,278; 4,795,454; 4,704,115; and 4,909,803; and U.S. Pat. Publ. No. 2009/0312730.

The chassis of the absorbent article may comprise suitable elasticized waistbands may be constructed in a number of different configurations including those described in U.S. Pat. Nos. 4,515,595 and 5,151,092.

Flaps may be integral with or joined to the chassis. The flaps may be permanently or refastenably joined to each other or to another edge of the chassis to form waist and leg openings. The flaps may be engaged to form said waist and leg openings when the absorbent articles are in the package - such that the articles are pre-closed or pre-fastened in the package.

Flaps may comprise fastening components. Some exemplary fastening components are disclosed in U.S. Patent Nos. 3,848,594; 4,662,875; 4,846,815; 4,894,060; 4,946,527; 5,151,092; 5,221,274; and 6,432,098. The fastening system may also include primary and secondary fastening components, as disclosed in U.S. Pat. No. 4,699,622.

The absorbent article may comprise "wings" that are intended to wrap the edges of the wearer's undergarments in the crotch region and/or affix the article to the undergarment to avoid poor folding and premature detachment. Exemplary absorbent articles comprising wings are disclosed in U.S. Pat. No. 8,039,685.

Various suitable belt-like flap configurations can be found in U.S. Pub. No. 2013-0211363.

The absorbent article may be a sanitary napkin. The sanitary napkin may comprise a liquid permeable topsheet, a liquid impermeable, or substantially liquid impermeable, backsheet, and an absorbent core positioned intermediate the topsheet and the backsheet. The absorbent core are described hereafter. In some forms, the sanitary napkin, may comprise a secondary topsheet instead and/or an acquisition layer(s). The sanitary napkin may comprise wings extending outwardly with respect to a longitudinal axis of the sanitary napkin. The sanitary napkin may also comprise a lateral axis. The wings may be joined to the topsheet, the backsheet, and/or the absorbent core. The sanitary napkin may also comprise a front edge, a rear edge longitudinally opposing the front edge, a first side edge, and a second side edge laterally opposing the first side edge. The longitudinal axis may extend from a midpoint of the front edge to a midpoint of the rear edge. The lateral axis may extend from a midpoint of the first side edge to a midpoint of the second side edge. The sanitary napkin may also be provided with additional features commonly found in sanitary napkins as is known in the art.

As shown, the topsheet and the backsheet have length and width dimensions generally larger than those of the absorbent core. The topsheet and the backsheet extend beyond the edges of the absorbent core to thereby form the periphery of the sanitary napkin. The topsheet, the backsheet, and the absorbent core may be assembled in a variety of well-known configurations know to those of skill in the art.

The absorbent core may be any absorbent member which is generally compressible, conformable, non-irritating to the wearer's skin, and capable of absorbing and retaining liquids such as urine and other certain body exudates. The absorbent core has a garment-facing side, a body-facing side, a pair of side edges, and a pair of end edges. The absorbent core may be manufactured in a wide variety of sizes and shapes (e.g., rectangular, hourglass, "T"-shaped, asymmetric, etc.) and from a wide variety of liquid-absorbent materials commonly used in sanitary napkins and other absorbent articles such as comminuted wood pulp which is generally referred to as airfelt. The absorbent core may comprise superabsorbent polymers (SAP) and less than 15%, less than 10%, less than 5%, less than 3%, or less than 1% of airfelt, or be completely free of airfelt. Examples of other suitable absorbent materials comprise creped cellulose wadding, meltblown polymers including coform, chemically stiffened, modified or cross-linked cellulosic fibers, tissue including tissue wraps and tissue laminates, absorbent foams, absorbent sponges, superabsorbent polymers, absorbent gelling materials, or any equivalent material or combinations of materials. In some forms, the absorbent core may comprise multiple layers of absorbent material. For example, in some forms, the absorbent core may comprise a first layer having a very high percentage of SAP, e.g. 100%, and be substantially free of airfelt. In such forms, a second layer may comprise a combination of airfelt and SAP in the percentage ranges provided above. Still in other forms, absorbent cores of the present invention may comprise high internal phase emulsion absorbent material which is known in the art.

The configuration and construction of the absorbent core may vary (e.g., the absorbent core may have varying caliper zones, a hydrophilic gradient, a superabsorbent gradient, or lower average density and lower average basis weight acquisition zones; or may comprise one or more layers or structures). Further, the size and absorbent capacity of the absorbent core may also be varied to accommodate wearers ranging from infants through adults. However, the total absorbent capacity of the absorbent core should be compatible with the design loading and the intended use of the sanitary napkin.

The absorbent core of the present disclosure may comprise one or more adhesives, for example, to help immobilize the SAP or other absorbent materials within a core wrap and/or to ensure integrity of the core wrap, in particular when the core wrap is made of two or more substrates. The core wrap may extend to a larger area than required for containing the absorbent material(s) within. Absorbent cores comprising relatively high amounts of SAP with various core designs are disclosed in U.S. Pat. No. 5,599,335 to Goldman et al., EP 1,447,066 to Busam et al., WO 95/11652 to Tanzer et al., U.S. Pat. Publ. No. 2008/0312622A1 to Hundorf et al., and WO 2012/052172 to Van Malderen.

The backsheet is positioned adjacent the garment-facing surface of the absorbent core and may be joined thereto by attachment methods (not shown) such as those well known in the art. For example, the backsheet may be secured to the absorbent core by a uniform continuous layer of adhesive, a patterned layer of adhesive, or an array of separate lines, spirals, or spots of adhesive. Alternatively, the attachment methods may comprise using heat bonds, pressure bonds, ultrasonic bonds, dynamic mechanical bonds, or any other suitable attachment methods or combinations of these attachment methods as are known in the art. Forms of the present disclosure are also contemplated wherein the absorbent core is not joined to the backsheet, the topsheet.

The backsheet may be impervious, or substantially impervious, to liquids (e.g., urine) and may be manufactured from a thin plastic film, although other flexible liquid impervious materials may also be used. As used herein, the term "flexible" refers to materials which are compliant and will readily conform to the general shape and contours of the human body. The backsheet may prevent, or at least inhibit, the exudates absorbed and contained in the absorbent core from wetting articles which contact the sanitary napkin such as bed sheets and undergarments, however, the backsheet may permit vapors to escape from the absorbent core (i.e., is breathable). Thus, the backsheet may comprise a polymeric film such as thermoplastic films of polyethylene or polypropylene. A suitable material for the backsheet is a thermoplastic film having a thickness of from about 0.012 mm (0.5 mil) to about 0.051 mm (2.0 mils), for example.

The topsheet is positioned adjacent the body-facing surface of the absorbent core and may be joined thereto and to the backsheet by attachment methods (not shown) such as those well known in the art. Suitable attachment methods are described with respect to joining the backsheet to the absorbent core. The topsheetand the backsheet may be joined directly to each other in the sanitary napkin periphery and may be indirectly joined together by directly joining them to the absorbent core by the attachment methods (not shown).

The topsheet may be compliant, soft feeling, and non-irritating to the wearer's skin. Further, the topsheet may be liquid pervious permitting liquids (e.g., urine) to readily penetrate through its thickness.

The consumer goods product can be a hair conditioning composition. The hair conditioning composition of the present invention typically comprises a cationic surfactant, high melting point fatty compound, and aqueous carrier.

The cationic surfactant can be included in the hair conditioning composition at a level of from about 1.0%, preferably from about 1.5%, more preferably from about 2.0%, still more preferably from about 3.0%, and to about 25%, preferably to about 10%, more preferably to about 8.0%, still more preferably to about 6.0% by weight of the hair conditioning composition, in view of providing the benefits of the present invention.

Preferably, in the present invention, the surfactant is water-insoluble. In the present invention, "water-insoluble surfactants" means that the surfactants have a solubility in water at 25°C of preferably below 0.5g/100g (excluding 0.5g/100g) water, more preferably 0.3g/100g water or less.

Cationic surfactant useful herein can be one cationic surfactant or a mixture of two or more cationic surfactants. Preferably, the cationic surfactant is selected from: mono-long alkyl quaternized ammonium salt; a combination of mono-long alkyl quaternized ammonium salt and di-long alkyl quaternized ammonium salt; mono-long alkyl amine; a combination of mono-long alkyl amine and di-long alkyl quaternized ammonium salt.

Cationic surfactant being a mono-long alkyl amine, more specifically, mono-long alkyl amidoamine may be preferred in view of improving its dry feel with the polyol and by the preparation method of the present invention.

Cationic surfactant being a mono-long alkyl quaternized ammonium salt may be preferred in view of improving its quick rinse feel with the polyol and by the preparation method of the present invention.

Cationic surfactant being either: a combination of mono-long alkyl quaternized ammonium salt and di-long alkyl quaternized ammonium salt; or a combination of mono-long alkyl amine and di-long alkyl quaternized ammonium salt, may be preferred in view of improving its dry feel such as less greasy and/or free flowing of hair (less clumping of hair), with the polyol and by the preparation method of the present invention.

Mono-long alkyl amine useful herein are those having one long alkyl chain of preferably from 12 to 30 carbon atoms, more preferably from 16 to 24 carbon atoms, still more preferably from 18 to 22 alkyl group. Mono-long alkyl amines useful herein also include mono-long alkyl amidoamines. Primary, secondary, and tertiary fatty amines are useful.

Particularly useful are tertiary amido amines having an alkyl group of from about 12 to about 22 carbons. Exemplary tertiary amido amines include: stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, diethylaminoethylstearamide. Useful amines in the present invention are disclosed in U.S. Patent 4,275,055, Nachtigal, et al.

These amines are used in combination with acids such as ℓ-glutamic acid, lactic acid, hydrochloric acid, malic acid, succinic acid, acetic acid, fumaric acid, tartaric acid, citric acid, ℓ-glutamic hydrochloride, maleic acid, and mixtures thereof; more preferably ℓ-glutamic acid, lactic acid, citric acid, at a molar ratio of the amine to the acid of from about 1 : 0.3 to about 1 : 2, more preferably from about 1 : 0.4 to about 1 : 1.

The mono-long alkyl quaternized ammonium salts useful herein are those having one long alkyl chain which has from 12 to 30 carbon atoms, preferably from 16 to 24 carbon atoms, more preferably C18-22 alkyl group. The remaining groups attached to nitrogen are independently selected from an alkyl group of from 1 to about 4 carbon atoms or an alkoxy, polyoxyalkylene, alkylamido, hydroxyalkyl, aryl or alkylaryl group having up to about 4 carbon atoms.

Mono-long alkyl quaternized ammonium salts useful herein are those having the formula (I): 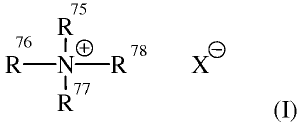 wherein one of R⁷⁵,R⁷⁶,R⁷⁷ and R⁷⁸ is selected from an alkyl group of from 12 to 30 carbon atoms or an aromatic, alkoxy, polyoxyalkylene, alkylamido, hydroxyalkyl, aryl or alkylaryl group having up to about 30 carbon atoms; the remainder of R⁷⁵, R⁷⁶, R⁷⁷ and R⁷⁸ are independently selected from an alkyl group of from 1 to about 4 carbon atoms or an alkoxy, polyoxyalkylene, alkylamido, hydroxyalkyl, aryl or alkylaryl group having up to about 4 carbon atoms; and X- is a salt-forming anion such as those selected from halogen, *(e.g.* chloride, bromide), acetate, citrate, lactate, glycolate, phosphate, nitrate, sulfonate, sulfate, alkylsulfate, and alkyl sulfonate radicals. The alkyl groups can contain, in addition to carbon and hydrogen atoms, ether and/or ester linkages, and other groups such as amino groups. The longer chain alkyl groups, e.g., those of about 12 carbons, or higher, can be saturated or unsaturated. Preferably, one of R⁷⁵, R⁷⁶, R⁷⁷ and R⁷⁸ is selected from an alkyl group of from 12 to 30 carbon atoms, more preferably from 16 to 24 carbon atoms, still more preferably from 18 to 22 carbon atoms, even more preferably 22 carbon atoms; the remainder of R⁷⁵, R⁷⁶, R⁷⁷ and R⁷⁸ are independently selected from CH₃, C₂H₅, C₂H₄OH, and mixtures thereof; and X is selected from the group consisting of Cl, Br, CH₃OSO₃, C₂H₅OSO₃, and mixtures thereof.

Nonlimiting examples of such mono-long alkyl quaternized ammonium salt cationic surfactants include: behenyl trimethyl ammonium salt; stearyl trimethyl ammonium salt; cetyl trimethyl ammonium salt; and hydrogenated tallow alkyl trimethyl ammonium salt.

When used, di-long alkyl quaternized ammonium salts are preferably combined with a mono-long alkyl quaternized ammonium salt or mono-long alkyl amine salt, at the weight ratio of from 1:1 to 1:5, more preferably from 1:1.2 to 1:5, still more preferably from 1:1.5 to 1:4, in view of stability in rheology and conditioning benefits.

Di-long alkyl quaternized ammonium salts useful herein are those having two long alkyl chains of from 12 to 30 carbon atoms, more preferably from 16 to 24 carbon atoms, still more preferably from 18 to 22 carbon atoms. Such di-long alkyl quaternized ammonium salts useful herein are those having the formula (I): 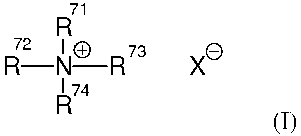 Wherein two of R⁷¹, R⁷², R⁷³ and R⁷⁴ are selected from an aliphatic group of from 12 to 30 carbon atoms, preferably from 16 to 24 carbon atoms, more preferably from 18 to 2 carbon atoms or an aromatic, alkoxy, polyxyalkylene, alkylamido, hydroxyalkyl, aryl or alkylaryl group having up to about 30 carbon atoms; the remainder of R⁷¹, R⁷², R⁷³ and R⁷⁴ are independently selected from an aliphatic group of from 1 to about 8 carbon atoms, preferably from 1 to 3 carbon atoms or an aromatic, alkoxy, polyoxyalkylene, alkylamido, hydroxyalkyl, aryl or alkylaryl group having up to about 8 carbon atoms; and X- is a salt-forming anion selected from the group consisting of halides such as chloride and bromide, C1-C4 alkyl sulfate such as methosulfate and ethosulfate, and mixtures thereof. The aliphatic groups can contain, in addition to carbon and hydrogen atoms, ether linkages, and other groups such as amino groups. The longer chain aliphatic groups, e.g., those of about 16 carbons, or higher, can be saturated or unsaturated. Preferably, two of R⁷¹, R⁷², R⁷³ and R⁷⁴ are selected from an alkyl group of from 12 to 30 carbon atoms, preferably from 16 to 24 carbon atoms, more preferably from 18 to 22 carbon atoms; and the remainder of R⁷¹, R⁷², R⁷³ and R⁷⁴ are independently selected from CH₃, C₂H₅, C₂H₄OH, CH₂C₆H₅, and mixtures thereof.

Such preferred di-long alkyl cationic surfactants include, for example, dialkyl (14-18) dimethyl ammonium chloride, ditallow alkyl dimethyl ammonium chloride, dihydrogenated tallow alkyl dimethyl ammonium chloride, distearyl dimethyl ammonium chloride, and dicetyl dimethyl ammonium chloride.

The high melting point fatty compound can be included in the hair conditioning composition at a level of from about 2.5%, preferably from about 3.0%, more preferably from about 4.0%, still more preferably from about 5.0%, and to about 30%, preferably to about 10%, more preferably to about 8.0% by weight of the hair conditioning composition, in view of providing the benefits of the present invention.

The high melting point fatty compound useful herein have a melting point of 25°C or higher, preferably 40°C or higher, more preferably 45°C or higher, still more preferably 50°C or higher, in view of stability of the emulsion especially the gel matrix. Preferably, such melting point is up to about 90°C, more preferably up to about 80°C, still more preferably up to about 70°C, even more preferably up to about 65°C, in view of easier manufacturing and easier emulsification. In the present invention, the high melting point fatty compound can be used as a single compound or as a blend or mixture of at least two high melting point fatty compounds. When used as such blend or mixture, the above melting point means the melting point of the blend or mixture.

The high melting point fatty compound useful herein is selected from the group consisting of fatty alcohols, fatty acids, fatty alcohol derivatives, fatty acid derivatives, and mixtures thereof. It is understood by the artisan that the compounds disclosed in this section of the specification can in some instances fall into more than one classification, e.g., some fatty alcohol derivatives can also be classified as fatty acid derivatives. However, a given classification is not intended to be a limitation on that particular compound, but is done so for convenience of classification and nomenclature. Further, it is understood by the artisan that, depending on the number and position of double bonds, and length and position of the branches, certain compounds having certain required carbon atoms may have a melting point of less than the above preferred in the present invention. Such compounds of low melting point are not intended to be included in this section. Nonlimiting examples of the high melting point compounds are found in International Cosmetic Ingredient Dictionary, Fifth Edition, 1993, and CTFA Cosmetic Ingredient Handbook, Second Edition, 1992.

Among a variety of high melting point fatty compounds, fatty alcohols are preferably used in the composition of the present invention. The fatty alcohols useful herein are those having from about 14 to about 30 carbon atoms, preferably from about 16 to about 22 carbon atoms. These fatty alcohols are saturated and can be straight or branched chain alcohols.

Preferred fatty alcohols include, for example, cetyl alcohol (having a melting point of about 56°C), stearyl alcohol (having a melting point of about 58-59°C), behenyl alcohol (having a melting point of about 71°C), and mixtures thereof. These compounds are known to have the above melting point. However, they often have lower melting points when supplied, since such supplied products are often mixtures of fatty alcohols having alkyl chain length distribution in which the main alkyl chain is cetyl, stearyl or behenyl group.

In the present invention, more preferred fatty alcohol is a mixture of cetyl alcohol and stearyl alcohol.

Generally, in the mixture, the weight ratio of cetyl alcohol to stearyl alcohol is preferably from about 1:9 to 9:1, more preferably from about 1:4 to about 4:1, still more preferably from about 1:2.3 to about 1.5:1

When using higher level of total cationic surfactant and high melting point fatty compounds, the mixture has the weight ratio of cetyl alcohol to stearyl alcohol of preferably from about 1:1 to about 4:1, more preferably from about 1:1 to about 2:1, still more preferably from about 1.2:1 to about 2:1, in view of avoiding to get too thick for spreadability. It may also provide more conditioning on damaged part of the hair.

The hair conditioning composition of the present invention preferably comprises an aqueous carrier. The level and species of the carrier are selected according to the compatibility with other components, and other desired characteristic of the product.

The carrier useful in the present invention includes water and water solutions of lower alkyl alcohols. The lower alkyl alcohols useful herein are monohydric alcohols having 1 to 6 carbons, more preferably ethanol and isopropanol.

Preferably, the aqueous carrier is substantially water. Deionized water is preferably used. Water from natural sources including mineral cations can also be used, depending on the desired characteristic of the product. Generally, the compositions of the present invention comprise from about 0% to about 99%, preferably from about 50% to about 95%, and more preferably from about 70% to about 90%, and more preferably from about 80% to about 90% water.

Preferably, in the present invention, the emulsion is in the form of a gel matrix. The gel matrix comprises the cationic surfactant system, the high melting point fatty compound, and an aqueous carrier. The gel matrix is suitable for providing various conditioning benefits, such as slippery feel during the application to wet hair and softness and moisturized feel on dry hair.

Preferably, when the gel matrix is formed, the cationic surfactant and the high melting point fatty compound are contained at a level such that the weight ratio of the cationic surfactant to the high melting point fatty compound is in the range of, preferably from about 1:1 to about 1:10, more preferably from about 1:1.5 to about 1:7, still more preferably from about 1:2 to about 1:6, in view of providing improved wet conditioning benefits.

Preferably, when the gel matrix is formed, the composition of the present invention is substantially free of anionic surfactants and anionic polymers, in view of stability of the gel matrix. In the present invention, "the composition being substantially free of anionic surfactants and anionic polymers" means that: the composition is free of anionic surfactants and anionic polymers; or, if the composition contains anionic surfactants and anionic polymers, the level of such anionic surfactants and anionic polymers is very low. In the present invention, a total level of such anionic surfactants and anionic polymers, if included, preferably 1% or less, more preferably 0.5% or less, still more preferably 0.1% or less by weight of the composition. Most preferably, the total level of such anionic surfactants and anionic polymers is 0% by weight of the composition.

The hair conditioning compositions of the present invention may further contain a silicone compound. It is believed that the silicone compound can provide smoothness and softness on dry hair. The silicone compounds herein can be used at levels by weight of the composition of preferably from about 0.1% to about 20%, more preferably from about 0.5% to about 10%, still more preferably from about 1% to about 8%.

Preferably, the silicone compounds have an average particle size of from about 1microns to about 50 microns, in the composition.

The silicone compounds useful herein, as a single compound, as a blend or mixture of at least two silicone compounds, or as a blend or mixture of at least one silicone compound and at least one solvent, have a viscosity of preferably from about 1,000 to about 2,000,000mPa·s at 25°C.

The viscosity can be measured by means of a glass capillary viscometer as set forth in Dow Corning Corporate Test Method CTM0004, July 20, 1970. Suitable silicone fluids include polyalkyl siloxanes, polyaryl siloxanes, polyalkylaryl siloxanes, polyether siloxane copolymers, amino substituted silicones, quaternized silicones, and mixtures thereof. Other nonvolatile silicone compounds having conditioning properties can also be used.

Preferred polyalkyl siloxanes include, for example, polydimethylsiloxane, polydiethylsiloxane, and polymethylphenylsiloxane. Polydimethylsiloxane, which is also known as dimethicone, is especially preferred.

The above polyalkylsiloxanes are available, for example, as a mixture with silicone compounds having a lower viscosity. Such mixtures have a viscosity of preferably from about 1,000mPa·s to about 100,000mPa·s, more preferably from about 5,000mPa·s to about 50,000mPa·s. Such mixtures preferably comprise: (i) a first silicone having a viscosity of from about 100,000mPa·s to about 30,000,000mPa·s at 25°C, preferably from about 100,000mPa·s to about 20,000,000mPa·s; and (ii) a second silicone having a viscosity of from about 5mPa·s to about 10,000mPa·s at 25°C, preferably from about 5mPa·s to about 5,000mPa·s. Such mixtures useful herein include, for example, a blend of dimethicone having a viscosity of 18,000,000mPa·s and dimethicone having a viscosity of 200mPa·s available from GE Toshiba, and a blend of dimethicone having a viscosity of 18,000,00OmPa·s and cyclopentasiloxane available from GE Toshiba.

The silicone compounds useful herein also include a silicone gum. The term "silicone gum", as used herein, means a polyorganosiloxane material having a viscosity at 25°C of greater than or equal to 1,000,000 centistokes. It is recognized that the silicone gums described herein can also have some overlap with the above-disclosed silicone compounds. This overlap is not intended as a limitation on any of these materials. The "silicone gums" will typically have a mass molecular weight in excess of about 200,000, generally between about 200,000 and about 1,000,000. Specific examples include polydimethylsiloxane, poly(dimethylsiloxane methylvinylsiloxane) copolymer, poly(dimethylsiloxane diphenylsiloxane methylvinylsiloxane) copolymer and mixtures thereof. The silicone gums are available, for example, as a mixture with silicone compounds having a lower viscosity. Such mixtures useful herein include, for example, Gum/Cyclomethicone blend available from Shin-Etsu.

Silicone compounds useful herein also include amino substituted materials. Preferred aminosilicones include, for example, those which conform to the general formula (I):

(R₁)ₐG₃₋ₐ-Si-(-OSiG₂)ₙ-(-OSiG_{b}(R₁)_{2-b})ₘ-O-SiG₃₋ₐ(R₁)ₐ

wherein G is hydrogen, phenyl, hydroxy, or C₁-C₈ alkyl, preferably methyl; a is 0 or an integer having a value from 1 to 3, preferably 1; b is 0, 1 or 2, preferably 1; n is a number from 0 to 1,999; m is an integer from 0 to 1,999; the sum of n and m is a number from 1 to 2,000; a and m are not both 0; R₁ is a monovalent radical conforming to the general formula CqH_{2q}L, wherein q is an integer having a value from 2 to 8 and L is selected from the following groups: -N(R₂)CH₂-CH₂-N(R₂)₂; -N(R₂)₂; -N(R₂)₃A⁻; -N(R₂)CH₂-CH₂-NR₂H₂A⁻; wherein R₂ is hydrogen, phenyl, benzyl, or a saturated hydrocarbon radical, preferably an alkyl radical from about C₁ to about C₂₀; A is a halide ion.

Highly preferred amino silicones are those corresponding to formula (I) wherein m=0, a=1, q=3, G=methyl, n is preferably from about 1500 to about 1700, more preferably about 1600; and L is -N(CH₃)₂ or -NH₂, more preferably -NH₂. Another highly preferred amino silicones are those corresponding to formula (I) wherein m=0, a=1, q=3, G=methyl, n is preferably from about 400 to about 600, more preferably about 500; and L is -N(CH₃)₂ or -NH₂, more preferably -NH₂. Such highly preferred amino silicones can be called as terminal aminosilicones, as one or both ends of the silicone chain are terminated by nitrogen containing group.

The above aminosilicones, when incorporated into the composition, can be mixed with solvent having a lower viscosity. Such solvents include, for example, polar or non-polar, volatile or non-volatile oils. Such oils include, for example, silicone oils, hydrocarbons, and esters. Among such a variety of solvents, preferred are those selected from the group consisting of non-polar, volatile hydrocarbons, volatile cyclic silicones, non-volatile linear silicones, and mixtures thereof. The non-volatile linear silicones useful herein are those having a viscosity of from about 1 to about 20,000 centistokes, preferably from about 20 to about 10,000 centistokes at 25°C. Among the preferred solvents, highly preferred are non-polar, volatile hydrocarbons, especially non-polar, volatile isoparaffins, in view of reducing the viscosity of the aminosilicones and providing improved hair conditioning benefits such as reduced friction on dry hair. Such mixtures have a viscosity of preferably from about 1,00mPa·s to about 100,000mPa·s, more preferably from about 5,000mPa·s to about 50,000mPa·s.

Other suitable alkylamino substituted silicone compounds include those having alkylamino substitutions as pendant groups of a silicone backbone. Highly preferred are those known as "amodimethicone". Commercially available amodimethicones useful herein include, for example, BY16-872 available from Dow Corning.

The silicone compounds may further be incorporated in the present composition in the form of an emulsion, wherein the emulsion is made my mechanical mixing, or in the stage of synthesis through emulsion polymerization, with or without the aid of a surfactant selected from anionic surfactants, nonionic surfactants, cationic surfactants, and mixtures thereof.

The hair conditioning composition of the present invention may include other additional components, which may be selected by the artisan according to the desired characteristics of the final product and which are suitable for rendering the composition more cosmetically or aesthetically acceptable or to provide them with additional usage benefits. Such other additional components generally are used individually at levels of from about 0.001% to about 10%, preferably up to about 5% by weight of the composition.

A wide variety of other additional components can be formulated into the present hair conditioning compositions. These include: other conditioning agents such as hydrolysed collagen with tradename Peptein 2000 available from Hormel, vitamin E with tradename Emix-d available from Eisai, panthenol available from Roche, panthenyl ethyl ether available from Roche, hydrolysed keratin, proteins, plant extracts, and nutrients; preservatives such as benzyl alcohol, methyl paraben, propyl paraben and imidazolidinyl urea; pH adjusting agents, such as citric acid, sodium citrate, succinic acid, phosphoric acid, sodium hydroxide, sodium carbonate; coloring agents, such as any of the FD&C or D&C dyes; perfumes; ultraviolet and infrared screening and absorbing agents such as benzophenones; and antidandruff agents such as zinc pyrithione, non-ionic surfactant such as mono-9-octadecanoate poly(oxy-1,2-ethanediyl) supplied as, for example, Tween 20.

The hair conditioning compositions of the present invention can be in the form of rinse-off products or leave-on products, and can be formulated in a wide variety of product forms, including but not limited to creams, gels, emulsions, mousses and sprays. The hair conditioning composition of the present invention is especially suitable for hair conditioners especially rinse-off hair conditioners.

**Composition:** The composition comprises or consists of chitin nanofibrils, lignin or its derivatives and at least one polymer or co-polymer. Said three components may be bound within a binary or ternary complex.

The composition preferably comprises from 0.2wt% and 1wt% chitin nanofibrils. The composition preferably comprises from 0.1w/w% to 5w/w% lignin or a derivative thereof, preferably the composition comprises from 0.1w/w% to less of 1% w/w lignin or a derivative thereof. The composition preferably comprises from 5w/w% to 15w/w% polymer or copolymer.

The liquid medium percentage in the compositions in the form of a solution/suspension is typically the balance to 100% (balance to 100%). Said solvent is preferably water.

The compositions of the invention can either be used as such in liquid form or else turned into a solid material.

The liquid forms are suspensions, dispersions or solutions in a suitable medium, optionally they can be thickened or gelled, so in the form of a gel too. The medium may be pure water or an aqueous or hydroalcoholic solution or an oily medium.

The compositions of the invention may be transformed into a solid material. For the purposes of the present application, the term "solid material" is not necessarily a solid, dehydrated and rigid material but a material of any solid, semi-solid, elastic, plastic or pasty consistency. Said material is for example in the form of non-woven fabric, film, foil, sheet, mono-layer or multi-layer membrane or paste.

The composition may be in solid form.

The composition may be in a form selected from: monolayer or multilayer non-woven fabric; monolayer or multilayer film; monolayer or multilayer foil; monolayer or multilayer sheet; monolayer or multilayer membrane; monolayer or multilayer paste; or any combination thereof.

**Chitin:** Within the present text, the term "chitin nanofibrils", "chitin nanocrystals" or simply "chitin" typically refers to chitin in its known form of nanometric fibrils with a thickness/diameter ranging between 2.8 and 25nm, and of 8nm and about 200nm in length (see WO-A-2006/048 829).

Although, due to their nanometric size chitin nanofibrils when dispersed in an aqueous medium produce a formulation with the appearance of a "solution", with this application the more accurate term nanofibrils "suspension" is used; this is because these nanofibrils are insoluble solids and therefore formally produce suspensions or dispersions. However, in the scope of the present application, the terms "nanofibrils suspension", "nanofibrils dispersion" or "nanofibrils solution" are considered equivalent and interchangeable.

For convenience chitin nanofibrils are typically formulated and used in a solution/suspension base containing about 2% (w/w) of nanofibrils in aqueous solution. The percentage of "chitin nanofibrils" indicated in this application therefore may refer to the percentage of said base solution/suspension within the chitin-lignin-polymer liquid mixture in a liquid medium.

Liquid mixtures of the three components usually contain about 30wt% of said 2w/w% chitin base solution. In absolute values therefore, the suspensions of the invention normally contain about 0.6wt% of chitin nanofibrils. Different percentages of base suspension can however be used for the preparation of the suspensions of the invention. In certain circumstances, for the purposes of the invention, mixtures containing between 10% and 50% (w/w) for example 15%, 20%, 25%, 30%, 35%, 40%, 45% of base suspension may also be used.

**Lignin:** The term "lignin" refers to an organic polyphenolic polymer consisting of three different monomers, i.e. cumarilic, coniferyl and sinapyl alcohol, in any of countless structures in which it is present in nature. It is known in fact that the amount of each constituent may vary depending, for example, on plant family or species. Given that lignin is widely described in the prior art it is not necessary to dwell here upon its structure and chemical/physical characteristics. Preferably, the lignin according to the invention is selected from lignin obtained from wood, preferably hard wood, such as the lignin from conifers, grass, wheat straw or mixtures thereof. As for its preparation, the lignin can be: kraft lignin, alkaline lignin, acetosolve lignin or steam exploded lignin. Regardless of its origin, lignin may also be raw lignin, lignin I, which is the purest fraction obtained from raw lignin or lignin II, which is the fraction of carboxylated lignin I. In a specific embodiment the lignin is obtained from wheat straw.

The lignin or a derivative thereof is preferably selected from: lignin derived from wood, grass and/or straw lignin; kraft lignin; alkaline lignin; acetosolve lignin; steam exploded lignin; raw lignin; lignin I; lignin II; or mixtures thereof.

Preferably, the lignin or a derivative thereof is selected from liginin having:
(a) a weight average molecular weight (Mw) in the range of from 12,300 Da to 14,300 Da, and a polydispersity in the range of from 3.1 to 2.5, and a degree of polymerization in the range of from 12 to 18; or
(b) a weight average molecular weight (Mw) in the range of from 2,300 Da to 4,300 Da, and a polydispersity in the range of from 2.6 to 4.6, and a degree of polymerization in the range of from 3 to 4; or
(c) a weight average molecular weight (Mw) in the range of from 6,300 Da to 8,100 Da, and a polydispersity in the range of from 2.3 to 3.1, and a degree of polymerization in the range of from 9 to 10.

The lignin content in the liquid mixtures of the invention is between 0.05% and 5% (w/w) of the mixture itself, for example 0.1%, 0.3%, 0.5%, 0, 8%, 1%, 2%, 3%, 4% or 5%. In a preferred embodiment the percentage of lignin is less than 1%, and most preferably between 0.1% and 0.5%.

Without wishing to limit the invention to particular theories, chitin nanofibrils and lignin appear to form complexes with each other by means of ester or ether bonds together with the involvement of lignin phenolic hydroxyls.

**Polymer or co-polymer:** Another component of the compositions and materials of the invention is the polymeric component, namely a polymer, a co-polymer or mixtures thereof. The function of this component is to confer together with the lignin the best mechanical properties to the materials and articles of the invention and in particular the consistency necessary, thus carrying out a substantially structural or thickening function.

Said polymeric component can be selected from the group comprising ethylene polyoxide (PEOX) at different molecular weights, for example, PEG from 400 to 8000, polyvinyl alcohol (PVA), PA 6 (Nylon 6), PUR (Poly Urethane), PES (Poli ethylene- Sulfide); natural biopolymers such as gelatin, cellulose or chitosan or polylactic acid and glycerol. Such a polymeric material may be in the form of organic fibres or nanofibres.

Preferably, said polymer or co-polymer is selected from: ethylene polyoxide; polylactic acid; polyglycol; polyvinyl alcohol; polyacrylate 6 (Nylon 6); polyurethane; polyethylene sulfur; gelatin; cellulose or chitosan; optionally in combination with inorganic micro/nanoparticles selected from micro/nanoparticles of: titanium dioxide; nanof silicon oxide; aluminum oxide (Al₂O₃); zinc oxide; zirconium dioxide; magnesium and aluminum oxide (MgAl₂O₄).

Optionally, in addition to the organic polymer component, the composition may comprise a polymer based on inorganic nanoparticles such as: Titanium dioxide, Silicon Oxide, Aluminium Oxide (Al2O3), Zinc Oxide, Zirconium Dioxide, Magnesium Oxide and Aluminium (MgAl2O4).

The polymer component content is preferably between 5% and 15% (w/w) of the liquid mixture, for example 6%, 7%, 8%, 9%, 10%, 11%, 12%, 13 %, 14%.

**Additional components:** The composition may also include one or more additional components selected from: vitamins, micronutrients (e.g. fluorine, selenium, iron, zinc, iodine), anti-inflammatory, antioxidant or anti-ageing substances, immunomodulating agents, enzymes, substances with a depigmentation action and metals.

The metals could be for example silver and/or bismuth and/or copper. In particular, the metals listed above may themselves form a complex with the chitin nanofibrils in any of the respective oxidation states or else in metallic form. For example, silver forms a complex with chitin nanofibrils in its elementary metallic state (oxidation state 0); bismuth can form a complex with chitin nanofibrils in its +2 or elementary oxidation state; copper can form a complex with chitin nanofibrils in its elementary or +1, +2 or oxidation states. In one embodiment of the invention the chitin nanofibrils form a complex with one or more of the metals Ag, Cu and/or Bi in their elemental states.

If the materials of the invention form complexes with the metals listed above, these complexes will feature antibacterial/antifungal activity.

The composition may comprises aloe vera, zinc, silicone including terminal amino silicone, shea butter, choline salicylate, zinc carbonate, terpene, limonene, monoethyleneamine, peroxide including di-acyl peroxide and/or di-benyl peroxide, silver, blood coaggulant, titanium dioxide, benzo triazole, sodium dichloroisocyanurate, perfume, sense aid, and any mixture thereof.

**Solid support:** Optionally the compositions or materials, in any of the liquid or solid physical forms indicated above is/are deposited upon a solid support which can be either soft, rigid or elastic. Preferably, the support is polymeric and selected from the group comprising: polypropylene, polyethylene, acrylate, collagen, gelatin, polylactate or other equivalent bio-compatible polymers. Alternatively, the support may be a fabric of variable thickness, a spongy material, or an absorbent matrix. In a specific embodiment of the invention, the material in suspension or solution, or even concentrate, is adsorbed onto an absorbent matrix.

The support can be a polymeric support selected from the group consisiting of: polypropylene; polyethylene; polyacrylate; collagen; gelatin; polylactate; polyvinyl alcohol; and combination thereof.

The support can be porous, preferably in the form of a fabric, sponge, foam and/or absorbent matrix.

Given that it confers structural strength to the non woven fabrics, films, films, pastes and gels, thus guaranteeing better consistency and also in view of subsequent processing or use, the function of the support, which is preferably polymeric, is substantially mechanical. The support is preferably selected from the group comprising: polypropylene, polyethylene, acrylate, collagen, gelatin, polylactate or other bio-compatible polymers. The support may alternatively be a fabric of variable thickness, a spongy material or an absorbent matrix. In a specific embodiment the support is polypropylene and/or polyethylene.

**Process for the preparation of the composition:** The composition can be prepared by a process comprising the following steps:
(a) preparing an aqueous suspension of chitin nanofibrils;
(b) preparing a basic aqueous solution of lignin or a derivative thereof;
(c) mixing the suspension of chitin nanofibrils and the solution of lignin or derivative thereof;
(d) adding to the obtained mixture at least one polymer or co-polymer;
(e) stirring the mixture comprising said chitin nanofibrils, said lignin or derivative thereof and said polymer or co-polymer, preferably for at least 48 hours; and optionally
(f) adding, in any of the above mentioned steps, one or more ingredients selected from the group: aloe vera, zinc, silicone including terminal amino silicone, shea butter, choline salicylate, zinc carbonate, terpene, limonene, monoethyleneamine, peroxide including di-acyl peroxide and/or di-benyl peroxide, silver, blood coaggulant, titanium dioxide, benzo triazole, sodium dichloroisocyanurate, perfume, sense aid, and any mixture thereof.

**Process for the preparation of chitin nanofibrils, lignin, and polymer liquid compositions:** The aqueous suspension of the substances according to the invention, presumably in the form of complexes, may be prepared according to a process comprising the following steps:
- preparing a first aqueous suspension of chitin nanofibrils;
- preparing a basic aqueous solution of lignin or derivative thereof;
- mixing the suspension of chitin nanofibrils and the solution of lignin or derivative thereof;
- adding at least one polymer or co-polymer material to the mixture obtained;
- stirring the mixture comprising said chitin nanofibrils, said lignin or derivative thereof and said polymer or co-polymer material for at least 48 hours.

In order to facilitate the dissolution of lignin, the aqueous solution containing it, can be adjusted to a basic pH by adding a base, for example NaOH, preferably at a concentration of 0.1 M.

The concentration of chitin nanofibrils, lignin, and the polymeric material in the respective suspensions or solutions is such to allow for the preparation of a material containing said components in the amounts and proportions described above.

In the embodiments wherein the suspension comprises one or more additional ingredients, as indicated above, the process provides that those components are added in any of the above steps. In particular, they may therefore be added: in the chitin suspension and/or in the lignin solution, or in the suspension obtained by mixing said first suspension and said solution or in the final suspension comprising the final chitin nanofibrils, lignin and the co-polymer. In particular, if silver, copper and bismuth in metallic form are added, the process of the invention includes a step of adding the metals Ag, Cu and/or Bi in salified ionic form and a reduction step of the same in the presence of chitin nanofibrils according to the following. The reduction step is preferably performed before the addition of the lignin.

The metal ions can be added for example, as nitrate, sulphate, chlorate or other usual Salts.

The metal ions reduction reaction can be carried out by a person skilled in the art according to any known method. For example, the reduction may be carried out in the presence of a reducing agent such as, glucose, starch, sucrose, fructose, ascorbic acid or any other natural reducing agent that is compatible with human physiology.

In particular, the reducing agent concentration can be between 0.1 and 2.5% (w/w). The reaction is carried out by stirring the aqueous suspension comprising the chitin nanofibrils and metal ions for a period of 5-10 minutes. This operation if preferably carried out at a temperature of between 20-25°C. The reducing agent is preferably glucose.

**Table 1 lists some example mixtures of the invention. The w/w percentages of the single components in the aqueous suspension are illustrated and the percentages of the single components net of the diluent (water) are in parentheses.**

| Film | content (w/w %) | | | |
|---|---|---|---|---|
| | PEOX % | Lignin % | Ulterior component | water |
| No. 1 | 7% powder | Wheat straw 0.1% powder | Chitin-nanofibrils 30% /v/v (2% suspension w/v) | 62.9% |
| No. 2 | 7% | Wheat straw 0.1% | Chitin-nanofibrils + Ag 30% | |
| No. 21 | 7% | Wheat straw 0.1% | Chitin-nanofibrils 30% | |
| No. 23 | 7% | Wheat straw 0.1% | Chitin-nanofibrils 30% collagen 0.1% | |
| No. 30 | 7% | WS ASOL 800 HEX 0.1% | Chitin-nanofibrils 30% | |
| No. 31 | 7% | WS F3(MeOH) 0.1% | Chitin nanofibrils 30% | |
| No. 34 | 7% | WS ASOL 800 HEX 0.5% | Chitin nanofibrils 30% | |

The composition can be prepared by a process comprising the following steps:
(a) preparing an aqueous suspension/solution comprising chitin nanofibrils, lignin or derivative thereof and at least one polymer or co-polymer and optionally, one or more ingredients selected from the group: aloe vera, zinc, silicone including terminal amino silicone, shea butter, choline salicylate, zinc carbonate, terpene, limonene, monoethyleneamine, peroxide including di-acyl peroxide and/or di-benyl peroxide, silver, blood coaggulant, titanium dioxide, benzo triazole, sodium dichloroisocyanurate, perfume, sense aid, and any mixture thereof; and
(b) transforming the aqueous suspension prepared in step (a) in a solid thin layer.

A "solid thin layer" means a sheet, a foil, a film, a film or a non-woven fabric etc., featuring variable thickness as needed. The thickness of said thin layer, regardless of the technique used, varies in the order of nanometres to millimetres, for example from 200nm to 5mm, preferably from 0.5 microns to 1mm, or from 1 micron to 0.5mm. For example, the thickness could be 200nm, 300nm, 500nm, 800nm, 1 micron, 10 microns, 50 microns, 100 microns, 800 microns, 1 millimetre, 5 millimetres.

The transformation of the suspension into a thin solid or paste layer or can be carried out according to any full or partial drying technique that is capable of transforming the suspension in accordance with what is indicated herein.

In a first embodiment of the invention, the technique used is electro spinning. In a second embodiment of the invention, the technique used is casting.

**Electrospinning:** Starting with the suspension as described above, electro spinning, also referred to as electrostatic spinning, results in filaments constituting or comprising chitin and lignin monofilaments. These filaments have a lower section diameter of 100nm and a length of several millimetres or even centimetres.

To this end electrospinning can be carried out using the NS LAB 500S equipment based on Nanospider Technology and produced and marketed by the Elmarco company (www.elmarco.com).

Advantageously, using the Nanospider Technology technique, a thin layer that is in the form of non-woven fabric is obtained.

Depending on the operating mode adopted, as will be apparent to a person skilled in the art, both a monolayer and multi-layer (with at least two thin layers) form of non-woven fabric can be obtained. In fact, transforming nanochitin and lignin suspensions with different compositions in sequence by means of electrospinning (suspensions comprising several additional components for example), it is possible to obtain multi-layer fabrics wherein each layer can present the same or different chemical and physical characteristics.

For the purposes of the present invention, the electrospinning can be carried out at a temperature between 18°C and 30°C at a voltage between 5 and 60kV, preferably between 5 and 45kV. Furthermore, depending on the operating conditions, the electrospinning may be carried out at a variable flow rate from ml/h to m3/h. In addition, the electrospinning may be conducted in an environment with a relative humidity of between 20-25%.

**Casting:** Casting is a technique that starting with a suspension allows a thin solid, semi-solid, plastic, pasty or gel layer to be obtained essentially in the form of a film, membrane, film or foil.

By way of example, casting may be carried out by distributing the suspension upon a suitable substrate of the invention in such a way as to have a suitable thickness, in the order of a few microns or a few millimetres for example. The distributed suspension is subsequently dried at a temperature of between 20 and 30°C for example, preferably at about 25°C.

The equipment used for the preparation of the matrix could be an MSK AFA L800 for example.

As in the case of electrospinning, casting also allows both monolayer and multilayer (with at least two superimposed thin layers) materials to be obtained. In addition, by depositing by means of casting suspensions with different compositions in sequence, it is possible to obtain multilayer materials with compositions/functions that are modulated by layer.

The process can optionally provide an additional step wherein the thin layer obtained by electrospinning or casting is deposited onto a solid support. Alternatively, during electrospinning or casting, the material is deposited directly onto said support according to the operating conditions of the various machines used (Laboratory or Industrial).

**Dimensions:** The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

**Documents:** Every document cited herein, including any cross referenced or related patent or application and any patent application or patent to which this application claims priority or benefit thereof, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

**Embodiments:** While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

### EXAMPLES

### Deposition studies:

The following actives in combination with the lignin/chitin composition were tested on the following substrates for the benefits described below:

| **Active** | **Substrate** | **Benefit** | **Output** |
|---|---|---|---|
| (A) Zinc Carbonate | Glass, Cermaic | Glass Damage | Deposition of Active |
| (B) Perfume | Fabric | Olfactive Benefits | |
| (C) Titanium dioxide | Glass, Ceramic | Cleaning of Windows | |

The following results from SEM EDAX were obtained:

| **Active** | **Substrate** | **Reference** | **Product** |
|---|---|---|---|
| Deposition of TiO2 | Glass | 0 Atomic % | 5.25 Atomic % |
| Deposition of TiO2 | Ceramic | 0 Atomic % | 0.39 Atomic% |
| Deposition of ZnCO3 | Glass | 0 Atomic% | 1.3 Atomic% |
| Deposition of ZnCO3 | Ceramic | 0 Atomic % | 9.4 Atomic% |

The following results from Primavera Scale were obtained:

| | **Damp Fabrics Freshness performance (primavera grade)** | **Damp Fabrics Freshness performance (primavera delta)** |
|---|---|---|
| **Reference** | 55 | Ref |
| **Product** | 97 | +42 |

The above results were obtained using the following test methods:

### Procedure for TiO₂ on Hard Surface:

### Ceramic:

**Preparation of Reference:** A 2.5x2.5 cm piece of a non-woven substrate is loaded with 0.005 g of TiO₂ which is dispersed in 0.5 mL of natural olive oil. The substrate is then folded and applied for 20 minutes on a small ceramic tile 2.5x2.5 cm which was previously wet with deionized water. The substrate is removed from the ceramic tile and analysed for TiO₂ deposited on the ceramic surface using SEM EDAX.

**Preparation of Product:** A 2.5x2.5 cm piece of the said substrate is loaded with 0.005 g of TiO₂ which is dispersed in 0.5 mL of natural olive oil. The substrate is then folded and applied for 20 minutes on a small ceramic tile 2.5x2.5 cm which was previously wet with deionized water. The substrate is removed from the ceramic tile and analysed for TiO₂ deposited on the ceramic surface using SEM EDAX.

### Glass:

**Preparation of Reference:** A 2.5x2.5 cm piece of a non-woven substrate is loaded with 0.005 g of TiO₂ which is dispersed in 0.5 mL of natural olive oil. The substrate is then folded and applied for 20 minutes on a glass slide 7.5x2.5 cm which was previously wet with deionized water. The substrate is removed from the glass slide and analysed for TiO₂ deposited on the glass slide using SEM EDAX.

**Preparation of Product:** A 2.5x2.5 cm piece of the said substrate is loaded with 0.005 g of TiO₂ which is dispersed in 0.5 mL of natural olive oil. The substrate is then folded and applied for 20 minutes on a glass slide 7.5x2.5 cm which was previously wet with deionized water. The substrate is removed from the glass slide and analysed for TiO₂ deposited on the glass slide using SEM EDAX.

### Procedure for ZnCO₃ on Hard Surface:

### Ceramic:

**Preparation of Reference:** A 2.5x2.5 cm piece of a non-woven substrate is loaded with 0.005 g of ZnCO₃ which is dispersed in 0.5 mL of natural olive oil. The substrate is then folded and applied for 20 minutes on a small ceramic tile 2.5x2.5 cm which was previously wet with deionized water. The substrate is removed from the ceramic tile and analysed for ZnCO₃ deposited on the ceramic surface using SEM EDAX.

**Preparation of Product:** A 2.5x2.5 cm piece of the said substrate is loaded with 0.005 g of ZnCO₃ which is dispersed in 0.5 mL of natural olive oil. The substrate is then folded and applied for 20 minutes on a small ceramic tile 2.5x2.5 cm which was previously wet with deionized water. The substrate is removed from the ceramic tile and analysed for ZnCO₃ deposited on the ceramic surface using SEM EDAX.

### Glass:

**Preparation of Reference:** A 2.5x2.5 cm piece of a non-woven substrate is loaded with 0.005 g of ZnCO₃ which is dispersed in 0.5 mL of natural olive oil. The substrate is then folded and applied for 20 minutes on a glass slide 7.5x2.5 cm which was previously wet with deionized water. The substrate is removed from the glass slide and analysed for ZnCO₃ deposited on the glass slide using SEM EDAX.

**Preparation of Product:** A 2.5x2.5 cm piece of the said substrate is loaded with 0.005 g of ZnCO₃ which is dispersed in 0.5 mL of natural olive oil. The substrate is then folded and applied for 20 minutes on a glass slide 7.5x2.5 cm which was previously wet with deionized water. The substrate is removed from the glass slide and analysed for ZnCO₃ deposited on the glass slide using SEM EDAX.

### Procedure for Perfume Deposition on Fabric:

**Preparation of Reference:** A 2.5x2.5 cm piece of a non porous cellulose substrate is loaded with 0.5 g of perfume. The substrate is then folded and applied for 20 minutes on a 3 x 1/8th Tonrose Towel 6.25cm x 12.5cm which was previously wet with deionized water. The substrate is removed from the fabric and the fabric is olfactively graded.

**Preparation of Product:** A 2.5x2.5 cm piece of the asid substrate is loaded with 0.5 g of perfume. The substrate is then folded and applied for 20 minutes on a 3 x 1/8th Tonrose Towel 6.25cm x 12.5cm which was previously wet with deionized water. The substrate is removed from the fabric and the fabric is olfactively graded.

**Freshness performance:** Panel grading is used to assess the freshness characteristics. The panelists are trained and calibrated and panel the fabrics versus the reference fabric using the following primavera scale where +2.5 indicates a meaningful but not consumer noticeable positive difference versus reference, +5.0 indicates a meaningful and consumer noticeable positive difference versus reference, and +7.5 indicates a meaningful and highly consumer noticeable positive difference versus reference. A difference of 2.5 is considered to be a technical difference on the primavera scale. Three replicates of fabrics were prepared for each sample, and each fabric is panelled by two different panelists.

### Illustrative examples:

**Shampoo Compositions:**

| **Ingredient** | **Wt. % Current Product** | **Wt. % New Product I** | **Wt. % New Product II** |
|---|---|---|---|
| Water | Balance | Balance | Balance |
| Cetyl Alcohol | 4.18% | 4.18% | 4.18% |
| Stearyl Alcohol | 7.52% | 7.52% | 7.52% |
| Sodium laureth-3 sulfate (28% Active) | 10.00% | 10.00% | 10.00% |
| 5-Chloro-2-methyl-4-isothiazolin-3-one, Kathon CG | 0.03% | 0% | 0% |
| Lignin | 0% | 0.2% | 0.7% |
| Chitin | 0% | 0.3% | 0.7% |

**Hair Conditioning:**

| **Components** | **Wt% Current Product I** | **Wt% Current Product II** | **Wt% New Product I** | **Wt% New Product II** |
|---|---|---|---|---|
| Behenyl trimethylammonium methosulfate | 2.97 | - | 2.97 | - |
| Stearamidopropyl dimethyl amine | - | 3.24 | - | 3.24 |
| Dicetyl dimethyl ammonium chloride | - | - | - | - |
| Cetyl alcohol | 1.01 | 4.25 | 1.01 | 4.25 |
| Stearyl alcohol | 2.53 | 2.93 | 2.53 | 2.93 |
| Benzyl alcohol | 0.4 | 0.4 | 0.4 | 0.4 |
| Deionized Water | Balance | Balance | Balance | Balance |
| L-glutamic acid | - | 1.04 | - | 1.04 |
| Preservative (Kathon CG) | 0.03 | 0.03 | 0 | 0 |
| Lignin | 0 | 0 | 0.2 | 0.7 |
| Chitin | 0 | 0 | 0.3 | 0.7 |
| Aminosilicone *3 | 1.5 | 1.5 | 1.5 | 1.5 |
| Perfume | 0.5 | 0.5 | 0.5 | 0.5 |

**Hand Dishwashing:**

| **Examples** | | | |
|---|---|---|---|
| | **Wt% Current Product I** | **Wt%** | **Wt%** |
| | | **New Product I** | **New Product II** |
| Alkyl ethoxy sulfate AExS | 16 | 16 | 16 |
| Amine oxide | 5.0 | 5.0 | 5.0 |
| C9-11 EO8 | 5 | 5 | 5 |
| GLDA | 0.7 | 0.7 | 0.7 |
| Solvent | 1.3 | 1.3 | 1.3 |
| Polypropylene glycol (Mn=2000) | 0.5 | 0.5 | 0.5 |
| Sodium chloride | 0.8 | 0.8 | 0.8 |
| Lignin | 0 | 0.2 | 0.7 |
| Chitin | 0 | 0.3 | 0.7 |
| Water | Balance | Balance | Balance |

**Granular laundry detergent compositions designed for front-loading automatic washing machines:**

| | **Wt**% **Current Product** | **Wt% New Product** | **Wt% New Product** |
|---|---|---|---|
| Linear alkylbenzenesulfonate | 8 | 8 | 8 |
| AE3S | 0 | 0 | 0 |
| C12-14 Alkylsulfate | 1 | 1 | 1 |
| AE7 | 2.2 | 2.2 | 2.2 |
| C₁₀₋₁₂ Dimethyl hydroxyethylammonium chloride | 0.75 | 0.75 | 0.75 |
| Crystalline layered silicate (δ-Na₂Si₂O₅) | 4.1 | 4.1 | 4.1 |
| Zeolite A | 5 | 5 | 5 |
| Citric Acid | 3 | 3 | 3 |
| Sodium Carbonate | 15 | 15 | 15 |
| Silicate 2R (SiO₂:Na₂O at ratio 2:1) | 0.08 | 0.08 | 0.08 |
| Soil release agent | 0.75 | 0.75 | 0.75 |
| Acrylic Acid/Maleic Acid Copolymer | 1.1 | 1.1 | 1.1 |
| Carboxymethylcellulose | 0.15 | 0.15 | 0.15 |
| Protease - Purafect® (84 mg active/g) | 0.2 | 0.2 | 0.2 |
| Amylase - Stainzyme Plus® (20 mg active/g) | 0.2 | 0.2 | 0.2 |
| Lipase - Lipex® (18.00 mg active/g) | 0.05 | 0.05 | 0.05 |
| Amylase - Natalase® (8.65 mg active/g) | 0.1 | 0.1 | 0.1 |
| Cellulase - Celluclean^{™} (15.6 mg active/g) | 0 | 0 | 0 |
| TAED | 3.6 | 3.6 | 3.6 |
| Percarbonate | 13 | 13 | 13 |
| Na salt of Ethylenediamine-N,N'-disuccinic acid, (S,S) isomer (EDDS) | 0.2 | 0.2 | 0.2 |
| Hydroxyethane di phosphonate (HEDP) | 0.2 | 0.2 | 0.2 |
| MgSO₄ | 0.42 | 0.42 | 0.42 |
| Perfume | 0.5 | 0.5 | 0.5 |
| Suds suppressor agglomerate | 0.05 | 0.05 | 0.05 |
| Soap | 0.45 | 0.45 | 0.45 |
| Sulphonated zinc phthalocyanine (active) | 0.0007 | 0.0007 | 0.0007 |
| S-ACMC | 0.01 | 0.01 | 0.01 |
| Direct Violet 9 (active) | 0 | 0 | 0 |
| Lignin | 0 | 0.2 | 0.7 |
| Chitin | 0 | 0.3 | 0.7 |
| Sulfate/ Water & Miscellaneous | Balance | Balance | Balance |

**Beauty Lotion/Cream:**

| | **Wt**% **Current Product** | **Wt% New Product I** | **Wt% New Product II** |
|---|---|---|---|
| Water | Balance | Balance | Balance |
| Glycerin | 7 | 7 | 7 |
| Disodium EDTA | 0.05 | 0.05 | 0.05 |
| Methylparaben | 0.1 | 0.1 | 0.1 |
| Sodium Dehydroacetate | 0.5 | 0.5 | 0.5 |
| Benzyl alcohol | 0.25 | 0.25 | 0.25 |
| GLW75CAP-MP (75% aq. TiO2 dispersion)¹ | 0.5 | 0.5 | 0.5 |
| Palmitoyl-dipeptide² | 0.0001 | 0.0001 | 0.0001 |
| N-acetyl glucosamine | 2 | 2 | 2 |
| Salicylic Acid | 1.5 | 1.5 | 1.5 |
| Isohexadecane | 3 | 3 | 3 |
| PPG15 Stearyl Ether | 4 | 4 | 4 |
| Isopropyl Isostearate | 1.3 | 1.3 | 1.3 |
| Sucrose polyester | 0.7 | 0.7 | 0.7 |
| Phytosterol | 0.5 | 0.5 | 0.5 |
| Cetyl alcohol | 0.4 | 0.4 | 0.4 |
| Stearyl alcohol | 0.5 | 0.5 | 0.5 |
| Behenyl alcohol | 0.4 | 0.4 | 0.4 |
| PEG-100 stearate | 0.1 | 0.1 | 0.1 |
| Cetearyl glucoside | 0.1 | 0.1 | 0.1 |
| Polyacrylamide/C13-14 | 2 | 2 | 2 |
| isoparaffin/laureth-7 | | | |
| Dimethicone/dimethiconol | 2 | 2 | 2 |
| Polymethylsilsequioxane | 0.25 | 0.25 | 0.25 |
| Lignin | 0 | 0.2 | 0.7 |
| Chitin | 0 | 0.3 | 0.7 |

**Personal Care product containing skin lightening:**

| **Component** | **Wt% Current Product** | **Wt% New Product I** | **Wt% New Product II** |
|---|---|---|---|
| Disodium EDTA | 0.100 | 0.100 | 0.100 |
| phlorogine | 2.000 | 0 | 0 |
| Phlorogine BG | 0 | 2.000 | 0 |
| deoxyArbutin | 0 | 0 | 2.000 |
| sucrose dilaurate | 0 | 0 | 0 |
| Bakuchiol | 0 | 0 | 0 |
| pyrenoine | 0 | 0 | 0 |
| Niacinamide | 5.000 | 5.000 | 5.000 |
| Isohexadecane | 3.000 | 3.000 | 3.000 |
| Isopropyl isostearate | 1.330 | 1.330 | 1.330 |
| Sucrose polycottonseedate | 0.670 | 0.670 | 0.670 |
| Polymethylsilsesquioxane | 0.250 | 0.250 | 0.250 |
| Cetearyl glucoside + cetearyl alcohol | 0.200 | 0.200 | 0.200 |
| Behenyl alcohol | 0.400 | 0.400 | 0.400 |
| Ethylparaben | 0.200 | 0.200 | 0.200 |
| Propylparaben | 0.100 | 0.100 | 0.100 |
| Cetyl alcohol | 0.320 | 0.320 | 0.320 |
| Stearyl alcohol | 0.480 | 0.480 | 0.480 |
| Tocopheryl acetate | 0.500 | 0.500 | 0.500 |
| PEG-100 stearate | 0.100 | 0.100 | 0.100 |
| Glycerin | 7.000 | 7.000 | 7.000 |
| Titanium dioxide | 0.604 | 0.604 | 0.604 |
| Polyacrylamide + C13-14 isoparaffin + laureth-7 | 2.000 | 2.000 | 2.000 |
| Panthenol | 1.000 | 1.000 | 1.000 |
| Benzyl alcohol | 0.400 | 0.400 | 0.400 |
| Dimethicone + dimethiconol | 2.000 | 2.000 | 2.000 |
| Lignin | 0 | 0.2 | 0.7 |
| Chitin | 0 | 0.3 | 0.7 |
| Water (to 100g) | Balance | Balance | Balance |

**Automatic Dishwashing Cleaning composition:**

| | **Powder (wt % based on 19 g portion)** | **Powder (wt% based on 19 g portion)** | **Powder (wt % based on 19 g portion)** |
|---|---|---|---|
| STPP | 34-38 | 34-38 | 34-38 |
| Alcosperse¹ | 7-12 | 7-12 | 7-12 |
| SLF-18 Polytergent² | 1-2 | 1-2 | 1-2 |
| Esterified substituted benzene sulfonate³ | 0.1-6.0 | 0.1-6.0 | 0.1-6.0 |
| Polymer⁴ | 0.2-6.0 | 0.2-6.0 | 0.2-6.0 |
| Sodium perborate monohydrate | 2-6 | 2-6 | 2-6 |
| Carbonate | 20-30 | 20-30 | 20-30 |
| 2.0r silicate | 5-9 | 5-9 | 5-9 |
| Sodium disilicate | 0-3 | 0-3 | 0-3 |
| Enzyme system⁵ | 0.1-5.0 | 0.1-5.0 | 0.1-5.0 |
| Pentaamine cobalt(III)chloride dichloride salt | 10-15 | 10-15 | 10-15 |
| TAED | 0-3 | 0-3 | 0-3 |
| Perfume, dyes, water and other components | Balance to 100% | Balance to 100% | Balance to 100% |

| | **Liquid (wt% based on 1.9 g portion)** | **Liquid (wt% based on 1.9 g portion)** | **Liquid (wt% based on 1.9 g portion)** |
|---|---|---|---|
| Dipropylene Glycol | 35-45 | 35-45 | 35-45 |
| SLF-19 Polytergent² | 40-50 | 40-50 | 40-50 |
| Neodol ® C11EO9 | 1-3 | 1-3 | 1-3 |
| Lignin | 0 | 0.2 | 0.7 |
| Chitin | 0 | 0.3 | 0.7 |
| Dyes, water and other components | Balance | Balance | Balance |

- 1: such as Alcosperse® 246 or 247, a sulfonated copolymer of acrylic acid from Alco Chemical Co.
- 2: linear alcohol ethoxylate from Olin Corporation
- 3: such as those described above
- 4: a sulfonated polymer such as those described above
- 5: one or more enzymes such as protease, mannaway, natalase, lipase and mixture thereof

### Determination of the regenerative and anti-inflammatory activity of the composition:

The substances to be tested were diluted in serum-free DMEM and those that after dissolving were basic (pH 9) were proceeded with bringing the pH value up to a physiological value of around 7. The solutions were subsequently sterilized by means of autoclaving. Those substances that instead were already present in solution were first sterilized in autoclave and subsequently brought up at the time of the experiment by a 1:100 dilution in DMEM to a physiological pH value. The "polypropylene support" sample is sterilised in an autoclave after being reduced to small sized fragments.

**Experimental Procedures:** The immortalised human keratinocyte cell line, HaCaT, was cultivated in DMEM to which - 10% fetal bovine serum (FBS) and pennicillina- streptomycin (100U/ml) was added at 37°C in an atmosphere of 5% CO₂. In a routine manner, the cells were plated within a 6-well tissue culture plate and used at 80% confluence. Before any treatment, in order to stop growth and to synchronize the cell culture, the semi-confluent monolayers were deprived of nourishment using serum-free DMEM.

Subsequently, in order to verify tissue regeneration activity, the anti-inflammatory and antibacterial activity of the substances tested, the cell cultures were treated (i) with the samples of the invention and with Lipopolysaccharides (LPA) from P. aeruginosa which is known for its ability to induce a strong inflammatory response to a concentration of 10pg/ml in this order; (ii) only with the samples of the invention. For the evaluation of the antibacterial activity, cultures of Pseudomonas a. and Staphylococcus were treated with systems Nos. I and VI.

A Real-Time PCR was carried out in order to assess:
- expression within cells treated with the Beta-defensin 2 (hBD-2) and metalloproteinases 2 and 9 (MMP-2 and -9) sample of the invention, which are involved in the tissue regeneration mechanisms;
- expression within cells treated simultaneously with the samples of the invention and with LPS, proinflammatory cytokines, particularly interleukin-8 (IL-8), interleukin-1 alpha (IL-1) and tumor necrosis factor alpha (TNF-alpha).

All of the treatments were performed in triplicate for 6 or 24 hours, with the only exception being that of polypropylene (reference) whose activity was assessed after 24 hours. At the end of the experiment the total RNA was isolated and 200ng used for reverse transcription into cDNA using commercial random hexamer primers at 42°C for 45 minutes as per the manufacturer's instructions. A Real time PCR was carried out using the LC Fast Start DNA Master SYBR Green kit using 2pl of cDNA which corresponds to 10ng of total RNA in a final volume of 20pl, 3mM MgCl₂ and 0.5pM of sense and antisense primer using commercial primers.

**List of Systems Tested:**

| **System** | **PEOX** | **Lignin** | **Chitin** | | | **Comments** |
|---|---|---|---|---|---|---|
| System No I | 7% | Lignin II 0.1 % | 30% | | | Sample weight: 389 mg. Diluted in 3.89ml of saline solution. |
| System No II | 7% | 0% | 30% | | | Sample weight: 370mg, diluted in 3.7ml of saline solution. |
| System No III | 7% | Lignin Aldrich-1% | 30% | | | Sample weight: 290mg, diluted in 2.9ml of saline solution to obtain a concentration of lignin equal to 1mg/ml. |
| System IV | 7% | Lignin Aldrich -5% | 30% | | | Sample weight: 260mg, diluted in 2.6ml of saline solution to obtain a concentration of lignin equal to 5mg/ml. |
| System V | 7% | Lignin Aldrich- 2.5% | 30% | | | Sample weight: 230mg, diluted in 2.3ml of saline solution to obtain a concentration of lignin equal to 2.5mg/ml. |
| System VI | 7% | Lignin I 0.1% | 30% | | Sample weight: 567mg. Diluted in 5.67ml of saline solution. | |
| System VII | 7% | Lignin I 0.1 % | 30% | | Sample weight: 420mg. Diluted in 4.2ml of saline solution. | |

| **Properties of certain lignin:** | | | | | | |
|---|---|---|---|---|---|---|
| | **Mn** | **Mw** | | **polydispersity** | | **Degree of polymerisation** |
| **Wheat Straw** | 3900±300 | 1300±1000 | | 3.3±0.2 | | 15 |
| **Lignin I** | 900±30 | 3300±1000 | | 3.6±1.0 | | 3-4 |
| **Lignin II** | 2600±50 | 7200±900 | | 2.7±0.4 | | 9-10 |

### RESULTS: Pro-inflammatory cytokines:

The results relating to the expression of the pro-inflammatory cytokines IL-8, IL-1alpha and INF-alpha in the cells treated with the sample Nos. I, II, III, IV and V are listed in the tables and illustrated in the block diagrams of Figures 1A, 1B, 1C. A reduction in the values of these markers is an indication of an anti-inflammatory activity. It should be noted that sample No. II is the comparison sample which does not contain lignin, while systems Nos. I, III, IV and V contain, respectively, 0.1%, 1%, 5% and 2.5% Lignin.

The results in Figure 1 show that system I containing low percentages of Lignin (0.1%) is able to greatly reduce the expression of pro-inflammatory cytokines (IL-8, IL-1alpha and TNF-alpha), while the samples containing higher percentages of lignin (III, IV and V) are less effective. It should be noted that the comparison sample No. II, which does not contain lignin, is not effective in significantly reducing the expression of cytokines in none of the tests.

### BRIEF DESCRIPTION OF THE FIGURES:

**Figure 1:** illustrates gene expression modulation in human cell lines of proinflammatory cytokines in HaCaT keratinocytes. The cell lines were treated with: control (CTR); with lipopolysaccharide (LPS) only; with samples of solution/suspension Nos. I, II, III, IV and V (as described in the examples). Figure 1A refers to interleukin 8; figure 1B to interleukin 1 alpha; figure 1C to tumour necrosis factor alpha.

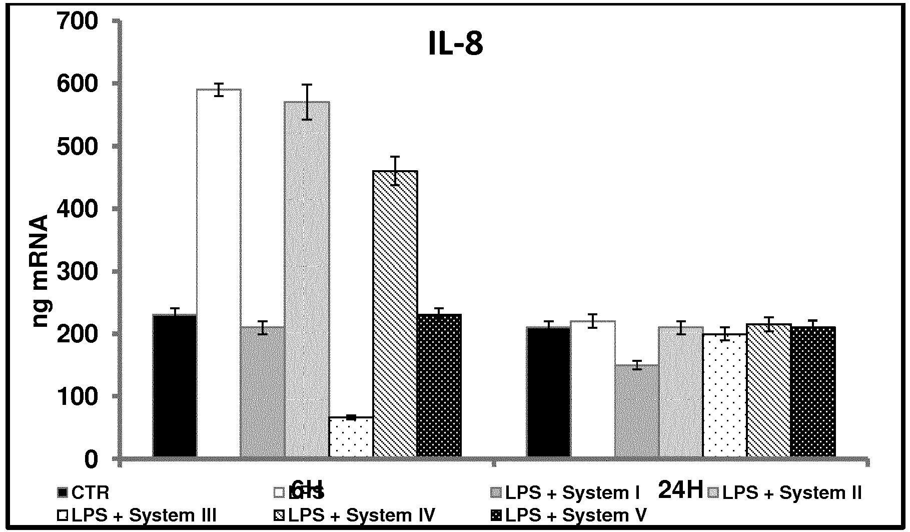

| | **IL-8** (**9ng mRNA**) | |
|---|---|---|
| **Systems** | **6H** | **24H** |
| **CTR** | 230±11 | 210±10 |
| **LPS** | 590±30 | 220±11 |
| **LPS + System I** | 210±10 | 150±7 |
| **LPS + System II** | 570±28 | 210±10 |
| **LPS + System III** | 67±3 | 200±10 |
| **LPS + System IV** | 460±23 | 215±11 |
| **LPS + System V** | 230±11 | 210±11 |

**Figure 1A:**

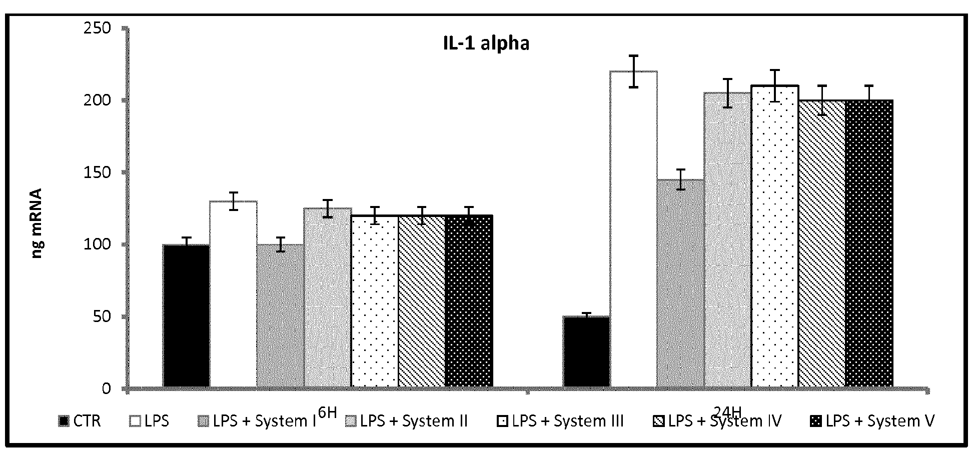

| | **IL-1 alpha (ng mRNA)** | |
|---|---|---|
| **Systems** | **6H** | **24H** |
| **CTR** | 100±5 | 50±2.5 |
| **LPS** | 130±6 | 220±11 |
| **LPS + System I** | 100±5 | 145±7 |
| **LPS + System II** | 125±6 | 205±10 |
| **LPS + System III** | 120±6 | 210±11 |
| **LPS + System IV** | 120±6 | 200±10 |
| **LPS + System V** | 120±6 | 200±10 |

**Figure 1B:**

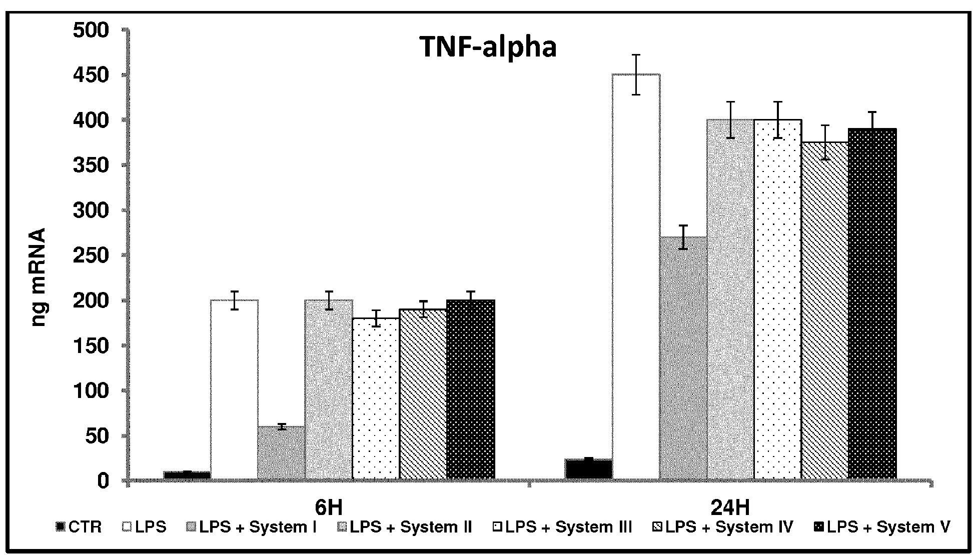

| | **TNF-alpha (ng mRNA)** | |
|---|---|---|
| **Systems** | **6H** | **24H** |
| **CTR** | 10±0.5 | 24±1 |
| **LPS** | 200±10 | 450±22 |
| **LPS + System I** | 60±3 | 270±13 |
| **LPS + System II** | 200±10 | 400±20 |
| **LPS + System III** | 180±9 | 400±20 |
| **LPS + System IV** | 190±9 | 375±19 |
| **LPS + System V** | 200±10 | 390±19 |

**Figure 1C:**

### RESULTS: Metalloproteinases and beta-defensin 2 (Tissue Repair Study):

Figures 2A, 2B and 2C refer instead to the expression of the metalloproteinases (MMP-2, MMP-9) and of the human beta-defensin 2 (hBD-2). An over-expression in the values of these markers is an indication of a tissue regeneration activity. All of the samples, Nos. I, III, IV and V were shown to have a positive effect in the tissue repair process mediated by induction of the hBD-2 expression (Fig. 2C), whereas with system No. II (not containing lignin) no activity was observed in this regard. As regards the MMP-2 and MMP-9 metalloproteinases expression induction tests, system No. I (containing 0.1% lignin) demonstrated high effectiveness in the induction of their expression, while samples containing high percentages of lignin did not demonstrate statistically significant and different activities from system II (not containing lignin). The high effectiveness of system No. I regarding MMP-2, MMP-9 and hDB2 expression induction was confirmed (data not reported here) in a parallel series of tests conducted with system Nos. VII, VI and I

### BRIEF DESCRIPTION OF THE FIGURES:

Figure 2 illustrates the modulation of the gene expression of metalloproteinases 2 (figure 2A) and 9 (figure 2b) and of the beta-defensin (figure 2C) in human keratinocytes HaCaT cell lines. The cell lines were treated with: control (CTR); with samples of solution/suspension Nos. I, II, III, IV and V (see examples).

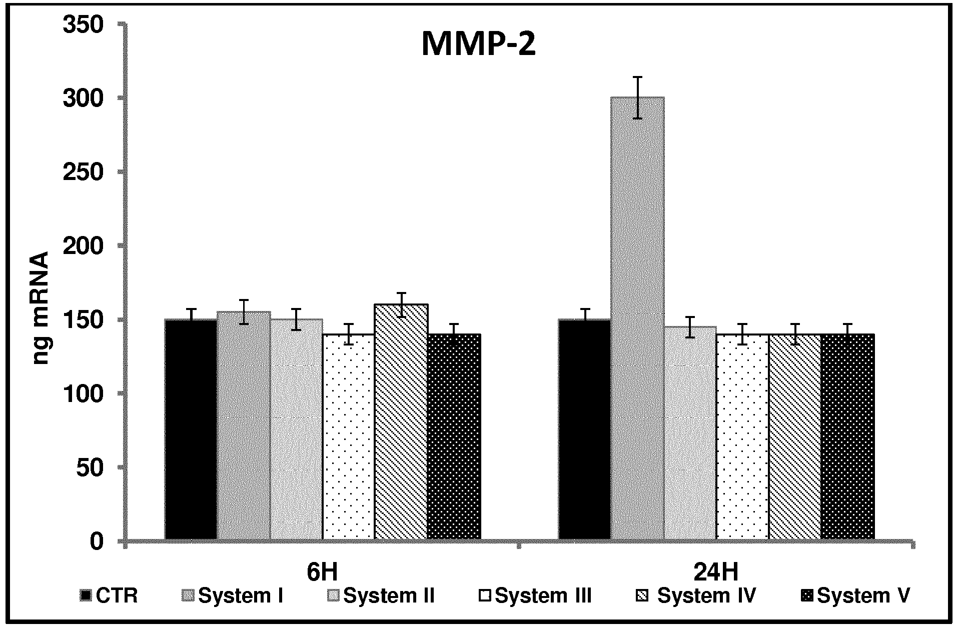

| | **MMP-2 (ng mRNA)** | |
|---|---|---|
| **Systems** | **6H** | **24H** |
| **CTR** | 150±7 | 150±7 |
| **System I** | 155±8 | 300±14 |
| **System II** | 150±7 | 145±7 |
| **System III** | 140±7 | 140±7 |
| **System IV** | 160±8 | 140±7 |
| **System V** | 140±7 | 140±7 |

**Figure 2A:**

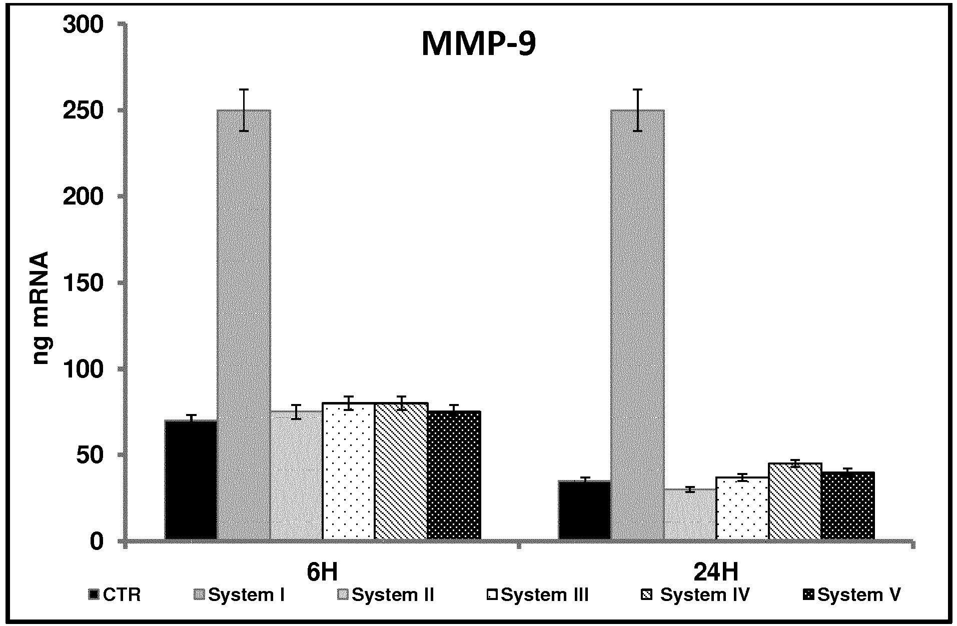

| | **MMP-9 (ng mRNA)** | |
|---|---|---|
| **Systems** | **6H** | **24H** |
| **CTR** | 70±3 | 35±2 |
| **System I** | 250±12 | 250±12 |
| **System II** | 75±4 | 30±1.5 |
| **System III** | 80±4 | 37±2 |
| **System IV** | 80±4 | 45±2 |
| **System V** | 75±4 | 40±2 |

**Figure 2B:**

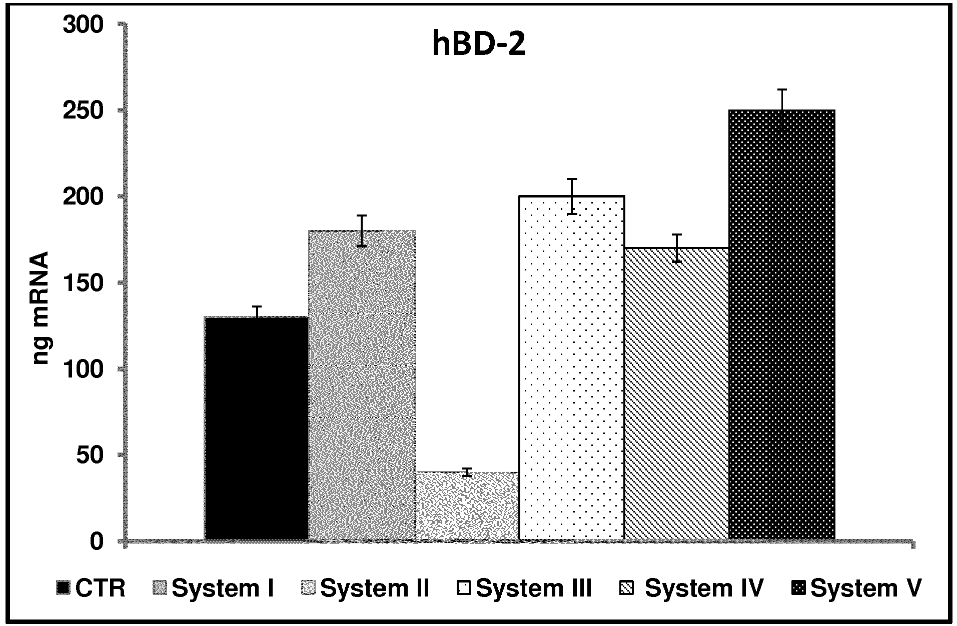

| **Systems** | **hBD-2 (ng mRNA**) |
|---|---|
| **CTR** | 130±6 |
| **System I** | 180±9 |
| **System II** | 40±2 |
| **System III** | 200±10 |
| **System IV** | 170±8 |
| **System V** | 250±12 |

**Figure 2C:**

### RESULTS: Pro-inflammatory cytokines induced by LPS

In another series of tests, the cells were treated with sample Nos. VII, VI and I and with the three samples containing the chitin nanofibrils and lignin complex only, but without a polymer. In parallel tests, the samples were tested both deposited on film and in liquid phase (solution/suspension). A reduction in the values of the pro-inflammatory cytokines is an indication of an anti-inflammatory activity. Note that the three samples, Nos. VII, VI and I respectively contain 0.1% raw Lignin, Lignin I, and Lignin II. The results are listed in the tables and illustrated in the block diagrams of Figures 3A, 3B and 3C.

The results in Figure 3 show that samples VII, VI and I, all containing 0.1% lignin, are capable, after 6 and 24 hours, of significantly decreasing the degree of expression of proinflammatory cytokines induced by LPS, and in a comparable manner. In addition, a comparison of the results obtained with the three samples shows that there are substantial differences in the pharmacological effect between the different types of lignin tested (Lignin I, Lignin II or raw lignin).

Results observed by the present inventors that are not reported here have also shown that the same level of efficiency regarding sample Nos. VII, VI and I was observed with samples containing only chitin nanofibrils and lignin. This confirms that the polymer or co-polymer present within the mixtures of the invention offers a merely mechanical function

### BRIEF DESCRIPTION OF THE FIGURES:

**Figure 3** illustrates gene expression modulation in human cell lines of proinflammatory cytokines in HaCaT keratinocytes. The cell lines were treated with: control (CTR); with lipopolysaccharide (LPS) only; with samples of solution/suspension Nos. VII, VI and I (compositions as described in the examples) or else with the same samples deposited on film. Figure 1A refers to interleukin 8; figure 1B to interleukin 1 alpha; figure 1C to tumour necrosis factor alpha.

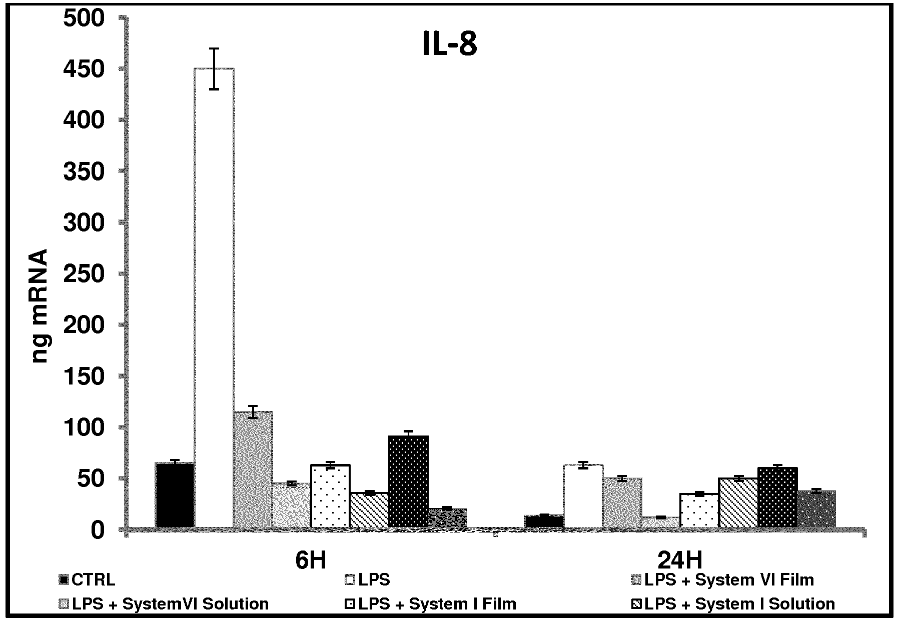

| | **IL-8 (ng mRNA)** | |
|---|---|---|
| **Systems** | **6H** | **24H** |
| **CTRL** | 65±3 | 14±1 |
| **LPS** | 450±20 | 63±3 |
| **LPS + System VI FILM** | 115±6 | 50±2.5 |
| **LPS + System VI** | 45±2 | 12±1 |
| **SOLUTION** | | |
| **LPS + System I FILM** | 63±3 | 35±2 |
| **LPS + System I** | 36±2 | 50±2.5 |
| **SOLUTION** | | |
| **LPS + System VII FILM** | 91±5 | 60±3 |
| **LPS + System VII** | 21±1 | 38±2 |
| **SOLUTION** | | |

**Figure 3A:**

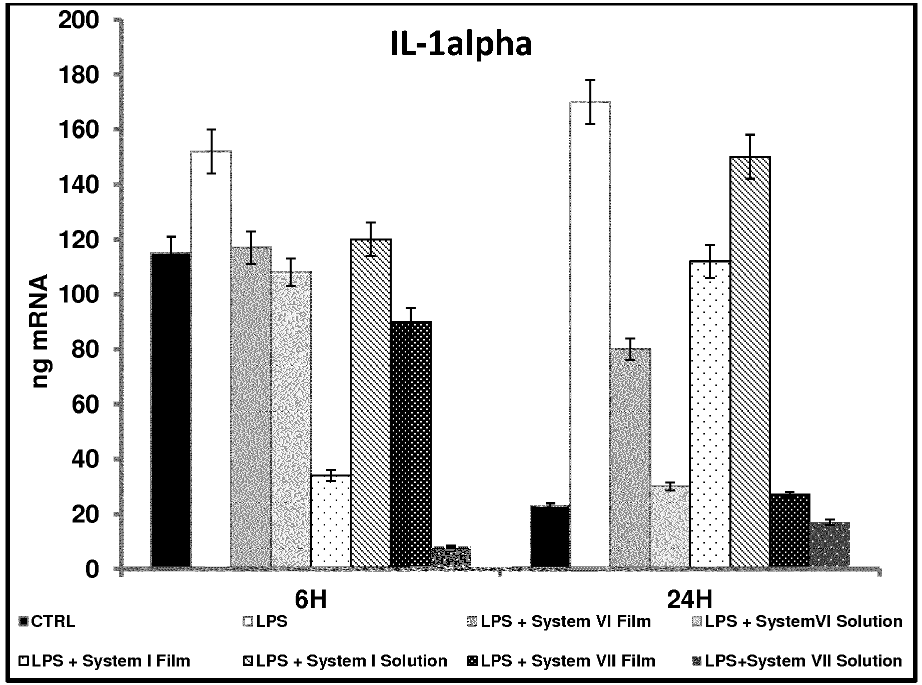

| | **IL-1 alpha** (**ng mRNA**) | |
|---|---|---|
| **Systems** | **6H** | **24H** |
| **CTRL** | 115±6 | 23±1 |
| **LPS** | 152±8 | 170±8 |
| **LPS + System VI FILM** | 117±6 | 80±4 |
| **LPS + System VI** | 108±5 | 30±1.5 |
| **SOLUTION** | | |
| **LPS + System I FILM** | 34±2 | 112±6 |
| **LPS + System I** | 120±6 | 150±8 |
| **SOLUTION** | | |
| **LPS + System VII FILM** | 90±5 | 60±3 |
| **LPS + System VII** | 8±0.5 | 17±1 |
| **SOLUTION** | | |

**Figure 3B:**

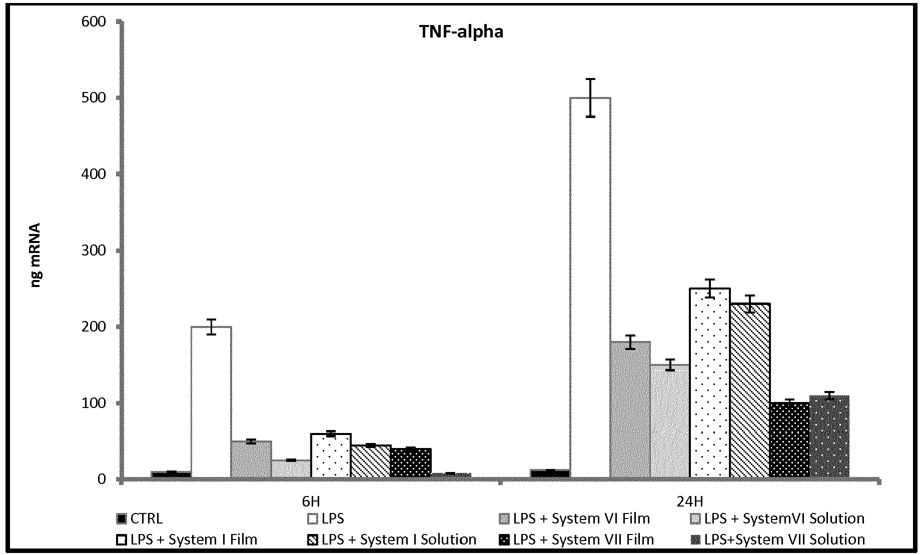

| | **TNF-alpha (ng mRNA)** | |
|---|---|---|
| **Systems** | **6H** | **24H** |
| **CTRL** | 10±0.5 | 12±0.5 |
| **LPS** | 200±10 | 500±25 |
| **LPS + System VI FILM** | 50±2.5 | 180±9 |
| **LPS + System VI** | 25±1 | 150±7 |
| **SOLUTION** | | |
| **LPS + System I FILM** | 60±3 | 250±12 |
| **LPS + System I** | 45±2 | 230±11 |
| **SOLUTION** | | |
| **LPS + System VII FILM** | 40±2 | 100±5 |
| **LPS + System VII** | 8±0.5 | 110±5 |
| **SOLUTION** | | |

**Figure 3C:**

## Claims

1. A consumer good product comprising:
(a) a composition comprising or consisting of a mixture of:
(i) chitin nanofibrils;
(ii) lignin or a derivative thereof; and
(iii) optionally, at least one polymer or co-polymer; and
(b) at least one consumer goods ingredient.

2. The consumer goods product according to claim 1, wherein the lignin or a derivative thereof is selected from: lignin derived from wood, grass and/or straw lignin; kraft lignin; alkaline lignin; acetosolve lignin; steam exploded lignin; raw lignin; lignin I; lignin II; or mixtures thereof.

3. The consumer goods product according to claim 1 or 2, wherein said polymer or copolymer is selected from: ethylene polyoxide; polylactic acid; polyglycol; polyvinyl alcohol; polyacrylate 6 (Nylon 6); polyurethane; polyethylene sulfur; gelatin; cellulose or chitosan; optionally in combination with inorganic micro/nanoparticles selected from micro/nanoparticles of: titanium dioxide; nanof silicon oxide; aluminum oxide (Al₂O₃); zinc oxide; zirconium dioxide; magnesium and aluminum oxide (MgAl₂O₄).

4. The consumer goods product according to any one of claims 1 to 3, wherein composition (a) is in form of a suspension, dispersion or solution in a liquid medium, optionally thickened or gelled.

5. The consumer goods product according to any one of claims 1 to 4, wherein composition comprises from 0.2wt% and 1wt% chitin nanofibrils.

6. The consumer goods product according to any one of claims 1 to 5, wherein composition (a) comprises from 0.1w/w% to 5w/w% lignin or a derivative thereof, preferably composition (a) comprises from 0.1w/w% to less of 1% w/w lignin or a derivative thereof.

7. The consumer goods product according to any one of claims 1 to 6, wherein composition (a) comprises from 5w/w% to 15w/w% polymer or copolymer.

8. The consumer goods product according to any one of claims 1 to 7, wherein composition (a) comprises aloe vera, zinc, silicone including terminal amino silicone, shea butter, choline salicylate, zinc carbonate, terpene, limonene, monoethyleneamine, peroxide including di-acyl peroxide and/or di-benyl peroxide, silver, blood coagulant, titanium dioxide, benzo triazole, sodium dichloroisocyanurate, perfume, sense aid, and any mixture thereof.

9. A consumer goods product according to any of claims 1-3 and 5-8, wherein composition (a) is in solid form.

10. A consumer goods product according to claim 9, wherein composition (a) is in a form selected from: monolayer or multilayer non-woven fabric; monolayer or multilayer film; monolayer or multilayer foil; monolayer or multilayer sheet; monolayer or multilayer membrane; monolayer or multilayer paste; or any combination thereof.

11. A process for the preparation of a consumer goods product accoding to any preceding claim, wherein composition (a) is prepared by a process comprising the following steps:
(a) preparing an aqueous suspension of chitin nanofibrils;
(b) preparing a basic aqueous solution of lignin or a derivative thereof;
(c) mixing the suspension of chitin nanofibrils and the solution of lignin or derivative thereof;
(d) adding to the obtained mixture at least one polymer or co-polymer;
(e) stirring the mixture comprising said chitin nanofibrils, said lignin or derivative thereof and said polymer or co-polymer, preferably for at least 48 hours; and optionally
(f) adding, in any of the above mentioned steps, one or more ingredients selected from the group: aloe vera, zinc, silicone including terminal amino silicone, shea butter, choline salicylate, zinc carbonate, terpene, limonene, monoethyleneamine, peroxide including di-acyl peroxide and/or di-benyl peroxide, silver, blood coagulant, titanium dioxide, benzo triazole, sodium dichloroisocyanurate, perfume, sense aid, and any mixture thereof.

12. The process of preparing a consumer goods product according to any of claims 9 and 10, wherein composition (a) is prepared by a process comprising the following steps:
(a) preparing an aqueous suspension/solution comprising chitin nanofibrils, lignin or derivative thereof and at least one polymer or co-polymer and optionally, one or more ingredients selected from the group: aloe vera, zinc, silicone including terminal amino silicone, shea butter, choline salicylate, zinc carbonate, terpene, limonene, monoethyleneamine, peroxide including di-acyl peroxide and/or di-benyl peroxide, silver, blood coaggulant, titanium dioxide, benzo triazole, sodium dichloroisocyanurate, perfume, sense aid, and any mixture thereof; and
(b) transforming the aqueous suspension prepared in step (a) in a solid thin layer.

13. The process according to claim 12, wherein said step of transformation is performed by casting or electro spinning.

14. A consumer goods product according to any of claims 1 to 10, wherein the composition
(a) is deposited or adsorbed on a support.

15. The consumer goods product according to claim 14, wherein said support is a polymeric support selected from the group consisiting of: polypropylene; polyethylene; polyacrylate; collagen; gelatin; polylactate; polyvinyl alcohol; and combination thereof.

16. The comsumer goods product according to claim 14 or 15, wherein the support is porous, preferably in the form of a fabric, sponge, foam and/or absorbent matrix.

17. A consumer goods product according to any of claims 1-10 and 14-16, wherein the consumer goods product is selected from: feminine pad; diaper; razor blade strip; hard surface cleaning sheet and/or wipe; and teeth treatment strip.

18. A consumer goods product according to claim 17, wherein composition (a) is in a form selected from: monolayer or multilayer non-woven fabric; monolayer or multilayer film; monolayer or multilayer foil; monolayer or multilayer sheet; monolayer or multilayer membrane; monolayer or multilayer paste; or any combination thereof.

19. A consumer goods product according to claims 18, wherein a consumer goods product comprises an ingredient selected from the group: aloe vera, zinc, silicone including terminal amino silicone, shea butter, choline salicylate, zinc carbonate, terpene, limonene, monoethyleneamine, peroxide including di-acyl peroxide and/or di-benyl peroxide, silver, blood coaggulant, titanium dioxide, benzo triazole, sodium dichloroisocyanurate, perfume, sense aid, and any mixture thereof, and
wherein composition (a) is in a form selected from: monolayer or multilayer non-woven fabric; monolayer or multilayer film; monolayer or multilayer foil; monolayer or multilayer sheet; monolayer or multilayer membrane; monolayer or multilayer paste; or any combination thereof, and wherein the ingredient is in direct physical contact with composition (a).

20. A consumer goods product according to any of claims 1-10 and 14-16, wherein the consumer goods product is selected from: skin cream; skin lotion; shaving preparation gel or foam; handwash laundry detergent; handwash dishwashing detergent; soap bar; liquid handwash soap; body wash; toothpaste; shampoo; and conditioner.

21. A consumer goods product according to claim 20, wherein composition (a) is in form of a suspension, dispersion or solution in a liquid medium, optionally thickened or gelled.

22. A consumer goods product according to any of claims 1-10 and 14-21, wherein the lignin or a derivative thereof is selected from liginin having:
(a) a weight average molecular weight (Mw) in the range of from 12,300 Da to 14,300 Da, and a polydispersity in the range of from 3.1 to 2.5, and a degree of polymerization in the range of from 12 to 18; or
(b) a weight average molecular weight (Mw) in the range of from 2,300 Da to 4,300 Da, and a polydispersity in the range of from 2.6 to 4.6, and a degree of polymerization in the range of from 3 to 4; or
(c) a weight average molecular weight (Mw) in the range of from 6,300 Da to 8,100 Da, and a polydispersity in the range of from 2.3 to 3.1, and a degree of polymerization in the range of from 9 to 10.

## Patentansprüche

1. Konsumgüterprodukt, umfassend:
(a) eine Zusammensetzung, umfassend oder bestehend aus einer Mischung aus:
(i) Chitin-Nanofibrillen;
(ii) Lignin oder einem Derivat davon; und
(iii) wahlweise, wenigstens einem Polymer oder Co-Polymer; und
(b) wenigstens einem Konsumgüterbestandteil.

2. Konsumgüterprodukt nach Anspruch 1, wobei das Lignin oder ein Derivat davon ausgewählt sind aus: Lignin aus Holz-, Gras- und/oder Stroh-Lignin; Kraftlignin; alkalisches Lignin; Acetosolv-Lignin; mit Dampf aufgebrochenes Lignin; Roh-Lignin; Lignin I, Lignin II; oder Mischungen davon.

3. Konsumgüterprodukt nach Anspruch 1 oder 2, wobei das Polymer oder Co-Polymer ausgewählt ist aus: Ethylenpolyoxid; polylaktische Säure; Polyglykol; Polyvinylalkohol; Polyacrylat 6 (Nylon 6); Polyurethan; Polyethylensulfid; Gelatine; Cellulose oder Chitosan; wahlweise in Verbindung mit anorganischen Mikro-/Nanopartikeln, ausgewählt aus Mikro-/Nanopartikeln von: Titandioxid; Nano-Siliziumoxid; Aluminiumoxid (Al₂O₃); Zinkoxid; Zirkoniumdioxid; Magnesium- und Aluminiumoxid (MgAl₂O₄).

4. Konsumgüterprodukt nach einem der Ansprüche 1 bis 3, wobei Zusammensetzung (a) in Form einer Suspension, Dispersion oder Lösung in einem flüssigen Medium, wahlweise verdickt oder geliert, ist.

5. Konsumgüterprodukt nach einem der Ansprüche 1 bis 4, wobei die Zusammensetzung zwischen 0,2 Gew.-% und 1 Gew.-% Chitin-Nanofibrillen umfasst.

6. Konsumgüterprodukt nach einem der Ansprüche 1 bis 5, wobei Zusammensetzung (a) von 0,1 Gew.-% bis 5 Gew.-% Lignin oder ein Derivat davon umfasst, vorzugsweise umfasst Zusammensetzung (a) von 0,1 Gew.-% bis weniger als 1 % Gew.-% Lignin oder ein Derivat davon.

7. Konsumgüterprodukt nach einem der Ansprüche 1 bis 6, wobei Zusammensetzung (a) zwischen 5 Gew.-% und 15 Gew.-% Polymer oder Co-Polymer umfasst.

8. Konsumgüterprodukt nach einem der Ansprüche 1 bis 7, wobei Zusammensetzung (a) Aloe Vera, Zink, Silikon einschließlich endständigem Aminosilikon, Sheabutter, Cholinsalicylat, Zinkcarbonat, Terpen, Limonen, Monoethanolamin, Peroxid einschließlich Diacylperoxid und/oder Dibenzoylperoxid, Silber, Blutkoagulat, Titandioxid, Benzo-Triazol, Natriumdichlorisocyanurat, Duftstoff, Wahrnehmungshilfe und Mischungen daraus umfasst.

9. Konsumgüterprodukt nach einem der Ansprüche 1 bis 3 und 5 bis 8, wobei Zusammensetzung (a) eine feste Form aufweist.

10. Konsumgüterprodukt nach Anspruch 9, wobei Zusammensetzung (a) eine Form ausgewählt aus Folgendem aufweist: einschichtiges oder mehrschichtiges Vliesmaterial; ein- oder mehrschichtiger Film; ein- oder mehrschichtige Folie; ein- oder mehrschichtige Lage; ein- oder mehrschichtige Membran; ein- oder mehrschichtige Paste; oder eine Kombination daraus.

11. Verfahren zur Herstellung eines Konsumgüterprodukts nach einem der vorstehenden Ansprüche, wobei Zusammensetzung (a) mittels eines Verfahrens vorbereitet wird, das die folgenden Schritte umfasst:
(a) Vorbereiten einer wässrigen Suspension aus Chitin-Nanofibrillen;
(b) Vorbereiten einer basischen wässrigen Lösung aus Lignin oder einem Derivat davon;
(c) Mischen der Suspension aus Chitin-Nanofibrillen und der Lösung aus Lignin oder einem Derivat davon;
(d) Hinzufügen wenigstens eines Polymers oder Co-Polymers zur erhaltenen Mischung;
(e) Verrühren der Mischung, umfassend die Chitin-Nanofibrillen, das Lignin oder Derivat davon und das Polymer oder Co-Polymer, vorzugsweise für wenigstens 48 Stunden; und wahlweise
(f) Hinzufügen, in einem der zuvor genannten Schritte, eines oder mehrerer Bestandteile, ausgewählt aus der Gruppe von: Aloe Vera, Zink, Silikon einschließlich endständigem Aminosilikon, Sheabutter, Cholinsalicylat, Zinkcarbonat, Terpen, Limonen, Monoethanolamin, Peroxid einschließlich Diacylperoxid und/oder Dibenzoylperoxid, Silber, Blutkoagulat, Titandioxid, Benzo-Triazol, Natriumdichlorisocyanurat, Duftstoff, Wahrnehmungshilfe und Mischungen daraus.

12. Verfahren zur Herstellung eines Konsumgüterprodukts nach einem der vorstehenden Ansprüche 9 und 10, wobei Zusammensetzung (a) mittels eines Verfahrens vorbereitet wird, das die folgenden Schritte umfasst:
(a) Vorbereiten einer wässrigen Suspension/Lösung, umfassend Chitin-Nanofibrillen, Lignin oder Derivate davon, und wenigstens ein Polymer oder Co-Polymer und wahlweise ein oder mehrere Bestandteile, ausgewählt aus der Gruppe von: Aloe Vera, Zink, Silikon einschließlich endständigem Aminosilikon, Sheabutter, Cholinsalicylat, Zinkcarbonat, Terpen, Limonen, Monoethanolamin, Peroxid einschließlich Diacylperoxid und/oder Dibenzoylperoxid, Silber, Blutkoagulat, Titandioxid, Benzo-Triazol, Natriumdichlorisocyanurat, Duftstoff, Wahrnehmungshilfe und Mischungen daraus; und
(b) Umwandeln der wässrigen Suspension, vorbereitet in Schritt (a) in eine massive dünne Schicht.

13. Verfahren nach Anspruch 12, wobei der Schritt des Umwandeins durch Gießen oder Elektrospinning durchgeführt wird.

14. Konsumgüterprodukt nach einem der Ansprüche 1 bis 10, wobei Zusammensetzung (a) auf einem Trägermedium angelagert oder adsorbiert wird.

15. Konsumgüterprodukt nach Anspruch 14, wobei das Trägermedium ein polymeres Trägermedium ist, ausgewählt aus der Gruppe bestehend aus: Polypropylen; Polyethylen; Polyacrylat; Kollagen; Gelatine; Polylactat; Polyvinylalkohol; und Kombination davon.

16. Konsumgüterprodukt nach Anspruch 14 oder 15, wobei das Trägermedium porös ist, vorzugsweise in der Form eines Stoffs, Schwamms, Schaumstoffs und/oder einer Absorptionsmatrix.

17. Konsumgüterprodukt nach einem der Ansprüche 1 bis 10 oder 14 bis 16, wobei das Konsumgüterprodukt ausgewählt ist aus: Damenbinde; Windel; Rasierklingenstreifen; Reinigungstuch/-lappen zum Reinigen harter Oberflächen; und Zahnpflegestreifen.

18. Konsumgüterprodukt nach Anspruch 17, wobei Zusammensetzung (a) eine Form ausgewählt aus Folgendem hat: einschichtiges oder mehrschichtiges Vliesmaterial; ein- oder mehrschichtiger Film; ein- oder mehrschichtige Folie; ein- oder mehrschichtige Lage; ein- oder mehrschichtige Membran; ein- oder mehrschichtige Paste; oder eine Kombination davon.

19. Konsumgüterprodukt nach Anspruch 18, wobei das Konsumgüterprodukt einen Bestandteil umfasst, ausgewählt aus folgender Gruppe: Aloe Vera, Zink, Silikon einschließlich endständigem Aminosilikon, Sheabutter, Cholinsalicylat, Zinkcarbonat, Terpen, Limonen, Monoethanolamin, Peroxid einschließlich Diacylperoxid und/oder Dibenzoylperoxid, Silber, Blutkoagulat, Titandioxid, Benzo-Triazol, Natriumdichlorisocyanurat, Duftstoff, Wahrnehmungshilfe und Mischungen davon, und
wobei Zusammensetzung (a) eine Form aufweist, die ausgewählt ist aus Folgendem: einschichtiges oder mehrschichtiges Vliesmaterial; ein- oder mehrschichtiger Film; ein- oder mehrschichtige Folie; ein- oder mehrschichtige Lage; ein- oder mehrschichtige Membran; ein- oder mehrschichtige Paste; oder eine Kombination davon,
und wobei der Bestandteil in direkten physischen Kontakt mit Zusammensetzung (a) kommt.

20. Konsumgüterprodukt nach einem der Ansprüche 1 bis 10 und 14 bis 16, wobei das Konsumgüterprodukt ausgewählt ist aus: Hautcreme; Hautlotion; Rasiergel oder -schaum; Hand-Wäschewaschmittel; Hand-Geschirrspülmittel, Seifenstück, Flüssig-Handwaschseife, Körperwaschmittel; Zahnpasta, Shampoo; und Conditioner.

21. Konsumgüterprodukt nach Anspruch 20, wobei Zusammensetzung (a) in Form einer Suspension, Dispersion oder Lösung in einem flüssigen Medium, wahlweise verdickt oder geliert, ist.

22. Konsumgüterprodukt nach einem der Ansprüche 1 bis 10 und 14 bis 21, wobei das Lignin oder ein Derivat davon aus einem Lignin mit folgenden Eigenschaften ausgewählt ist:
(a) ein durchschnittliches Molekulargewicht (Gewichtsmittel) im Bereich von 12.300 Da bis 14.300 Da, und einer Polydispersität im Bereich von 3,1 bis 2,5, und einem Polymerisationsgrad im Bereich von 12 bis 18; oder
(b) ein durchschnittliches Molekulargewicht (Gewichtsmittel) im Bereich von 2.300 Da bis 4.300 Da, und einer Polydispersität im Bereich von 2,6 bis 4,6, und einem Polymerisationsgrad im Bereich von 3 bis 4; oder
(b) ein durchschnittliches Molekulargewicht (Gewichtsmittel) im Bereich von 6.300 Da bis 8.100 Da, und einer Polydispersität im Bereich von 2,3 bis 3,1, und einem Polymerisationsgrad im Bereich von 9 bis 10.

## Revendications

1. Produit de biens de consommation comprenant :
(a) une composition comprenant ou constituée d'un mélange de :
(i) nanofibrilles de chitine ;
(ii) lignine ou un dérivé de celle-ci ; et
(iii) éventuellement, au moins un polymère ou copolymère ; et
(b) au moins un ingrédient de biens de consommation.

2. Produit de biens de consommation selon la revendication 1, dans lequel la lignine ou un dérivé de celle-ci est choisi parmi : de la lignine dérivée de lignine de bois, d'herbe et/ou de paille ; de la lignine kraft ; de la lignine alcaline ; de la lignine acétosolve ; de la lignine fragmentée par vapeur ; de la lignine brute ; de la lignine I ; de la lignine II ; ou des mélanges de ceux-ci.

3. Produit de biens de consommation selon la revendication 1 ou 2, dans lequel ledit polymère ou copolymère est choisi parmi : le polyoxyde d'éthylène ; l'acide polylactique ; le polyglycol ; l'alcool polyvinylique ; le polyacrylate 6 (Nylon 6) ; le polyuréthane ; le polyéthylène soufré ; la gélatine ; la cellulose ou le chitosane ; éventuellement en combinaison avec des micro/nanoparticules inorganiques choisies parmi des micro/nanoparticules de : dioxyde de titane ; nano-oxyde de silicium ; oxyde d'aluminium (Al₂O₃) ; oxyde de zinc ; dioxyde de zirconium ; oxyde de magnésium et d'aluminium (MgAl₂O₄).

4. Produit de biens de consommation selon l'une quelconque des revendications 1 à 3, dans lequel la composition (a) est sous la forme d'une suspension, d'une dispersion ou d'une solution dans un milieu liquide, éventuellement épaissie ou gélifiée.

5. Produit de biens de consommation selon l'une quelconque des revendications 1 à 4, dans lequel la composition comprend de 0,2 % en poids à 1 % en poids de nanofibrilles de chitine.

6. Produit de biens de consommation selon l'une quelconque des revendications 1 à 5, dans lequel la composition (a) comprend de 0,1 % en poids/poids à 5 % en poids/poids de lignine ou d'un dérivé de celle-ci, de préférence la composition (a) comprend de 0,1 % en poids/poids à moins de 1 % en poids/poids de lignine ou d'un dérivé de celle-ci.

7. Produit de biens de consommation selon l'une quelconque des revendications 1 à 6, dans lequel la composition (a) comprend de 5 % en poids/poids à 15 % en poids/poids de polymère ou copolymère.

8. Produit de biens de consommation selon l'une quelconque des revendications 1 à 7, dans lequel la composition (a) comprend de l'aloe vera, du zinc, de la silicone y compris l'aminosilicone terminale, du beurre de karité, du salicylate de choline, du carbonate de zinc, du terpène, du limonène, de la monoéthylèneamine, du peroxyde y compris du peroxyde de diacyle et/ou peroxyde de dibenzoyle, de l'argent, du coagulant sanguin, du dioxyde de titane, du benzotriazole, du dichloroisocyanurate de sodium, du parfum, de l'auxiliaire sensoriel, et l'un quelconque mélange de ceux-ci.

9. Produit de biens de consommation selon l'une quelconque des revendications 1 à 3 et 5 à 8, dans lequel la composition (a) est sous forme solide.

10. Produit de biens de consommation selon la revendication 9, dans lequel la composition (a) est sous une forme choisie parmi : un textile non tissé monocouche ou multicouche ; un film monocouche ou multicouche ; une feuille métallique monocouche ou multicouche ; une feuille monocouche ou multicouche ; une membrane monocouche ou multicouche ; une pâte monocouche ou multicouche ; ou n'importe quelle combinaison de ceux-ci.

11. Procédé de préparation d'un produit de biens de consommation selon l'une quelconque des revendications précédentes, dans lequel la composition (a) est préparée par un procédé comprenant les étapes suivantes :
(a) préparation d'une suspension aqueuse de nanofibrilles de chitine ;
(b) préparation d'une solution aqueuse basique de lignine ou d'un dérivé de celle-ci ;
(c) mélange de la suspension de nanofibrilles de chitine et de la solution de lignine ou d'un dérivé de celle-ci ;
(d) ajout au mélange obtenu d'au moins un polymère ou copolymère ;
(e) agitation du mélange comprenant lesdites nanofibrilles de chitine, ladite lignine ou ledit dérivé de celle-ci et ledit polymère ou copolymère, de préférence pendant au moins 48 heures ; et facultativement
(f) l'ajout, dans n'importe laquelle des étapes susmentionnées, d'un ou plusieurs ingrédients choisis parmi le groupe suivant : de l'aloe vera, du zinc, de la silicone y compris de l'aminosilicone terminale, du beurre de karité, du salicylate de choline, du carbonate de zinc, du terpène, du limonène, de la monoéthylèneamine, du peroxyde y compris du peroxyde de diacyle et/ou peroxyde de dibenzoyle, de l'argent, du coagulant sanguin, du dioxyde de titane, du benzotriazole, du dichloroisocyanurate de sodium, du parfum, de l'auxiliaire sensoriel, et l'un quelconque mélange de ceux-ci.

12. Procédé de préparation d'un produit de biens de consommation selon l'une quelconque des revendications 9 et 10, dans lequel la composition (a) est préparée par un procédé comprenant les étapes suivantes :
(a) préparation d'une suspension/solution aqueuse comprenant des nanofibrilles de chitine, de la lignine ou un dérivé de celle-ci et au moins un polymère ou copolymère et éventuellement, un ou plusieurs ingrédients choisis parmi le groupe suivant : de l'aloe vera, du zinc, de la silicone y compris de l'aminosilicone terminale, du beurre de karité, du salicylate de choline, du carbonate de zinc, du terpène, du limonène, de la monoéthylèneamine, du peroxyde y compris du peroxyde de diacyle et/ou peroxyde de dibenzoyle, de l'argent, du coagulant sanguin, du dioxyde de titane, du benzotriazole, du dichloroisocyanurate de sodium, du parfum, de l'auxiliaire sensoriel, et l'un quelconque mélange de ceux-ci ; et
(b) transformation de la suspension aqueuse préparée à l'étape (a) en une couche mince solide.

13. Procédé selon la revendication 12, dans lequel ladite étape de transformation est effectuée par coulage ou filage électrostatique.

14. Produit de biens de consommation selon l'une quelconque des revendications 1 à 10, dans lequel la composition (a) est déposée ou adsorbée sur un support.

15. Produit de biens de consommation selon la revendication 14, dans lequel ledit support est un support polymère choisi parmi le groupe constitué de :
polypropylène ; polyéthylène ; polyacrylate ; collagène ; gélatine ; polylactate ; alcool polyvinylique ; et une combinaison de ceux-ci.

16. Produit de biens de consommation selon la revendication 14 ou 15, dans lequel le support est poreux, de préférence sous la forme d'un tissu, d'une éponge, de mousse et/ou d'une matrice absorbante.

17. Produit de biens de consommation selon l'une quelconque des revendications 1 à 10 et 14 à 16, où le produit de biens de consommation est choisi parmi : une serviette hygiénique féminine ; une couche ; une bande de lame de rasoir ; une feuille et/ou lingette de nettoyage des surfaces dures ; et une bande de traitement dentaire.

18. Produit de biens de consommation selon la revendication 17, dans lequel la composition (a) est sous une forme choisie parmi : un textile non tissé monocouche ou multicouche ; un film monocouche ou multicouche ; une feuille métallique monocouche ou multicouche ; une feuille monocouche ou multicouche ; une membrane monocouche ou multicouche ; une pâte monocouche ou multicouche ; ou l'une quelconque combinaison de ceux-ci.

19. Produit de biens de consommation selon les revendications 18, où le produit de biens de consommation comprend un ingrédient choisi parmi le groupe suivant : de l'aloe vera, du zinc, de la silicone y compris de l'aminosilicone terminale, du beurre de karité, du salicylate de choline, du carbonate de zinc, du terpène, du limonène, de la monoéthylèneamine, du peroxyde y compris du peroxyde de diacyle et/ou peroxyde de dibenzoyle, de l'argent, du coagulant sanguin, du dioxyde de titane, du benzotriazole, du dichloroisocyanurate de sodium, du parfum, de l'auxiliaire sensoriel, et l'un quelconque mélange de ceux-ci, et
dans lequel la composition (a) est sous une forme choisie parmi : un textile non tissé monocouche ou multicouche ; un film monocouche ou multicouche ; une feuille métallique monocouche ou multicouche ; une feuille monocouche ou multicouche ; une membrane monocouche ou multicouche ; une pâte monocouche ou multicouche ; ou l'une quelconque combinaison de ceux-ci,
et dans lequel l'ingrédient est en contact physique direct avec la composition (a).

20. Produit de biens de consommation selon l'une quelconque des revendications 1 à 10 et 14 à 16, où le produit de biens de consommation est choisi parmi : une crème pour la peau ; une lotion pour la peau ; un gel ou une mousse de préparation au rasage ;
un détergent pour le lavage du linge à la main ; un détergent de lavage de la vaisselle à la main ; un pain de savon ; un savon liquide de lavage des mains ; un produit de lavage pour le corps ; une pâte dentifrice ; du shampooing ; et un conditionneur.

21. Produit de biens de consommation selon la revendication 20, dans lequel la composition (a) est sous la forme d'une suspension, d'une dispersion ou d'une solution dans un milieu liquide, éventuellement épaissie ou gélifiée.

22. Produit de biens de consommation selon l'une quelconque des revendications 1 à 10 et 14 à 21, dans lequel la lignine ou un dérivé de celle-ci est choisi parmi de la lignine possédant :
(a) une masse moléculaire moyenne en poids (Mw) dans la plage allant de 12 300 Da à 14 300 Da, et une polydispersité dans la plage allant de 3,1 à 2,5, et un degré de polymérisation dans la plage allant de 12 à 18 ; ou
(b) une masse moléculaire moyenne en poids (Mw) dans la plage allant de 2300 Da à 4300 Da, et une polydispersité dans la plage allant de 2,6 à 4,6, et un degré de polymérisation dans la plage allant de 3 à 4 ; ou
(c) une masse moléculaire moyenne en poids (Mw) dans la plage allant de 6300 Da à 8100 Da, et une polydispersité dans la plage allant de 2,3 à 3,1, et un degré de polymérisation dans la plage allant de 9 à 10.
